# EUROPEAN PATENT APPLICATION

(11) **EP 4 455 163 A1**
(43) Date of publication of application: **30.10.2024**
(21) Application number: 22911263.6
(22) Date of filing: 21.12.2022
(51) Int. Cl.: C07K 16/28, A61K 39/395, A61P 17/00, A61P 37/08, B01J 20/281, C12N 15/13, C12P 21/08, G01N 30/02, G01N 30/88

(54) **ANTIBODY VARIANT WITH REDUCED BIOLOGICAL ACTIVITY**

(30) Priority: 22.12.2021 JP 2021207714
(71) Applicant: CHUGAI SEIYAKU KABUSHIKI KAISHA, Tokyo 115-8543 (JP)
(72) Inventor: HOSOGUCHI, Kensaku, Tokyo 115-8543 (JP); HARA, Ai, Tokyo 115-8543 (JP); MASUDA, Souta, Tokyo 115-8543 (JP); TAKAGI, Misato, Tokyo 115-8543 (JP); NAMBU, Shusuke, Tokyo 115-8543 (JP); SATO, Kumi, Tokyo 115-8543 (JP)
(74) Representative: Vossius & Partner Patentanwälte Rechtsanwälte mbB
(86) International application number: PCT/JP2022/047035
(87) International publication number: WO 2023/120561

(57) **Abstract**

In one non-limiting embodiment, the present disclosure provides product variants of nemolizumab (antibody variants), particularly product variants with lower biological activity than nemolizumab. The present disclosure also provides nemolizumab-containing compositions that comprise the variants, methods for reducing the variants, and methods for analyzing the variants.

## Description

### [Technical Field]

The present invention relates to antibody variants of nemolizumab, for example, antibody variants with low biological activity as compared to nemolizumab. The present invention also relates to pharmaceutical compositions with a low content of such antibody variants. The present invention further relates to methods for detecting such antibody variants and methods for analyzing such antibody variants.

### [Background Art]

Antibodies are drawing attention as pharmaceuticals because of their high stability in the plasma and less side effects. In particular, there are many IgG type antibody pharmaceuticals on the market, and also a large number of antibody pharmaceuticals currently under development. Generally, antibody pharmaceuticals contain, in addition to a desired substance (antibody) as an active ingredient, various components including product variants (antibody variants) derived from the desired substance. Such product variants are categorized into product-related substances which have efficacy and safety comparable to the desired substance, and product-related impurities which do not have efficacy and safety comparable to the desired substance. Some variants are generated at varying rates depending on the conditions of the process of producing the drug substance. Because of this heterogeneity present in antibody pharmaceuticals, it is required to set release specifications and shelf-life specifications for drug substances and drug products with respect to each component, and control the quality of antibody pharmaceuticals.

In recent years, monoclonal antibodies that bind to interleukin-31 (IL-31) receptor A (IL-31RA) and have a function of inhibiting the binding of IL-31 to IL-31A were found (Patent Literatures (PTLs) 1-3). Of these, the anti-IL-31RA antibody nemolizumab (CIM331) acts as an IL-31 antagonist and inhibits the function of IL-31, which is considered a pruritus-inducing cytokine. It was thus subjected to a clinical trial targeting atopic dermatitis patients, and its results were recently reported (Non-Patent Literature (NPL) 1). However, there has been no report of whether antibody variants with reduced biological activity compared to nemolizumab antibody are generated in the process of producing drug substances and drug products of nemolizumab, and what percentages of such variants they contain.

### [Citation List]

### [Patent Literature]

[PTL 1] WO2007/142325
[PTL 2] WO2009/072604
[PTL 3] WO2010/064697

### [Non-Patent Literature]

[NPL 1] N Engl J Med 2020; 383:141-150

### [Summary of Invention]

### [Technical Problem]

The present invention was made in view of the above-mentioned circumstances. An objective of the present invention is to provide product variants (antibody variants) of nemolizumab, nemolizumab-containing compositions that contain such antibody variants and methods for producing such compositions, methods for reducing such antibody variants, and methods for analyzing such antibody variants.

### [Solution to Problem]

As a result of dedicated studies to achieve the above objective, the present inventors succeeded in identifying antibody variants (product variants of nemolizumab) contained in pharmaceutical compositions that contain nemolizumab as an active ingredient. The inventors also found that these antibody variants include those with low biological activity (activity of inhibiting IL-31 signaling by binding to an IL-31 receptor) compared to nemolizumab. Further, the inventors found methods for detecting and analyzing the antibody variants efficiently.

The present disclosure is based on these findings and, in one non-limiting embodiment, relates to the following:
[1] A variant of an antibody having a variable region comprising the amino acid sequence DGYDDGPYTLET (SEQ ID NO: 3), wherein
   (a) the amino acid residue D at position 1 from the N-terminus of the above sequence,
   (b) the amino acid residue D at position 4 from the N-terminus of the above sequence, or (c) the amino acid residue D at position 5 from the N-terminus of the above sequence is a succinimide residue or an isoaspartic acid residue.
[2] The antibody variant of [1], wherein the sequence is a CDR sequence.
[2.2] The antibody variant of [1], wherein the sequence is a CDR3 sequence.
[3] The antibody variant of [1], wherein the sequence is a sequence contained in a heavy chain.
[4] The antibody variant of [1], wherein the antibody having a variable region comprising the amino acid sequence DGYDDGPYTLET (SEQ ID NO: 3) is:
   (1) an antibody comprising a heavy chain variable region comprising CDR1 set forth in SEQ ID NO: 1, CDR2 set forth in SEQ ID NO: 2, and CDR3 set forth in SEQ ID NO: 3, and a light chain variable region comprising CDR1 set forth in SEQ ID NO: 4, CDR2 set forth in SEQ ID NO: 5, and CDR3 set forth in SEQ ID NO: 6;
   (2) an antibody comprising a heavy chain variable region set forth in SEQ ID NO: 7 and a light chain variable region set forth in SEQ ID NO: 8; or
   (3) an antibody comprising a heavy chain set forth in SEQ ID NO: 9 and a light chain set forth in SEQ ID NO: 10.
[5] The antibody variant of [1], wherein the antibody having a variable region comprising the amino acid sequence DGYDDGPYTLET (SEQ ID NO: 3) is nemolizumab.
[6] The antibody variant of any one of [1]-[5], which has a shorter retention time than the antibody having a variable region comprising the amino acid sequence DGYDDGPYTLET (SEQ ID NO: 3) when separated using anion exchange chromatography.
[7] The antibody variant of any one of [1]-[6], which has lower biological activity than the antibody having a variable region comprising the amino acid sequence DGYDDGPYTLET (SEQ ID NO: 3).
[1.2.0] A pharmaceutical composition comprising the antibody variant of any one of [1]-[7].
[1.2.1] A pharmaceutical composition comprising the antibody variant of any one of [1]-[7], wherein the ratio of an isoaspartic acid residue-containing antibody variant in the pharmaceutical composition to the sum of the antibody variant and the unmodified antibody is 36% or less, and/or, after enzymatic digestion, the ratio of an isoaspartic acid residue-containing peptide derived from the antibody variant to the sum of the isoaspartic acid residue-containing peptide and the corresponding unmodified peptide is 20% or less.
[1.2.2] A pharmaceutical composition comprising the antibody variant of any one of [1]-[7], wherein the ratio of an isoaspartic acid residue-containing antibody variant in the pharmaceutical composition to the sum of the antibody variant and the unmodified antibody is 27.8% or less, and/or, after enzymatic digestion, the ratio of an isoaspartic acid residue-containing peptide derived from the antibody variant to the sum of the isoaspartic acid residue-containing peptide and the corresponding unmodified peptide is 15% or less.
[1.2.3] A pharmaceutical composition comprising the antibody variant of any one of [1][7], wherein the ratio of an isoaspartic acid residue-containing antibody variant in the pharmaceutical composition to the sum of the antibody variant and the unmodified antibody is 17.9% or less, and/or, after enzymatic digestion, the ratio of an isoaspartic acid residue-containing peptide derived from the antibody variant to the sum of the isoaspartic acid residue-containing peptide and the corresponding unmodified peptide is 9.4% or less.
[1.2.4] A pharmaceutical composition comprising the antibody variant of any one of [1]-[7], wherein when the pharmaceutical composition is separated using anion exchange chromatography, the ratio of the peak area containing an isoaspartic acid residue-containing antibody variant to the total peak area is 40.3 area% or less, 30.4 area% or less, or 19.3 area% or less.
[1.2.5] The pharmaceutical composition of any one of [1.2.1]-[1.2.4], which comprises nemolizumab.
[1.2.6] A pharmaceutical composition comprising the antibody variant of any one of [1]-[7], wherein the ratio of a succinimide residue-containing antibody variant in the pharmaceutical composition to the sum of the antibody variant and unmodified antibody is 36% or less, and/or, after enzymatic digestion, the ratio of a succinimide residue-containing peptide derived from the antibody variant to the sum of the succinimide residue-containing peptide and the corresponding unmodified peptide is 20% or less.
[1.2.7] A pharmaceutical composition comprising the antibody variant of any one of [1]-[7], wherein the ratio of a succinimide residue-containing antibody variant in the pharmaceutical composition to the sum of the antibody variant and unmodified antibody is 27.8% or less, and/or, after enzymatic digestion, the ratio of a succinimide residue-containing peptide derived from the antibody variant to the sum of the succinimide residue-containing peptide and the corresponding unmodified peptide is 15% or less.
[1.2.8] A pharmaceutical composition comprising the antibody variant of any one of [1]-[7], wherein the ratio of a succinimide residue-containing antibody variant in the pharmaceutical composition to the sum of the antibody variant and unmodified antibody is 18.7% or less, and/or, after enzymatic digestion, the ratio of a succinimide residue-containing peptide derived from the antibody variant, to the sum of the succinimide residue-containing peptide and the corresponding unmodified peptide is 9.8% or less.
[1.2.9] A pharmaceutical composition comprising the antibody variant of any one of [1]-[7], wherein when the pharmaceutical composition is separated using anion exchange chromatography, the ratio of the peak area containing a succinimide residue-containing antibody variant to the total peak area is 29.1 area% or less, 21.6 area% or less, or 13.8 area% or less.
[1.2.10] The pharmaceutical composition of any one of [1.2.6]-[1.2.9], which comprises nemolizumab.
[1.2.11] The pharmaceutical composition of any one of [1.2.0]-[1.2.9], which is for use in either or both of treatment and prevention of at least one of atopic dermatitis, dialysis pruritus, and other pruritus.
[1.2.12] A method for treating or preventing at least one of atopic dermatitis, dialysis pruritus, and other pruritus, comprising administering the pharmaceutical composition of any one of [1.2.0]-[1.2.9].
[1.3.1] A method for detecting the antibody variant of any one of [1]-[7], comprising the step of separating a sample comprising an antibody having a variable region comprising the amino acid sequence DGYDDGPYTLET (SEQ ID NO: 3) or the antibody that has been enzymatically treated, by affinity chromatography, ion exchange chromatography, normal-phase chromatography, reverse-phase chromatography, hydrophilic interaction chromatography (HILIC), hydrophobic interaction chromatography (HIC), charge-based separation, size exclusion chromatography (SEC), gel permeation chromatography (GPC), or a combination thereof.
[1.3.2] The method of [1.3.1], wherein the ion exchange chromatography is anion exchange chromatography.
[1.3.3] The method of [1.3.2], wherein the anion exchange chromatography uses a column with an inner diameter of 4.6-7.5 mm, a length of 35-150 mm, and a particle diameter of 2.5-10 µm at a column temperature of 25-50°C and performs elution with a mobile phase of pH6.0-10.0.
[1.3.4] The method of [1.3.2] or [1.3.3], wherein the anion exchange chromatography uses a column with an inner diameter of 7.5 mm, a length of 75 mm, and a particle diameter of 2.5 µm at a column temperature of 40°C and performs elution with a mobile phase of pH7.5 comprising 250 mmol/L sodium chloride.
[1.3.5] The method of any one of [1.3.1]-[1.3.4], which comprises the step of quantifying a peptide by mass spectrometry and/or UV absorption measurement, following the step of separating a sample comprising the antibody that has been enzymatically treated.
[1.3.6] The method of any one of [1.3.1]-[1.3.5], wherein the antibody variant of any one of [1]-[7] is used as a reference material (standard).
[1.4.1] A method for producing a composition comprising nemolizumab, comprising the step of performing the method of any one of [1.3.1]-[1.3.6].
[1.4.2] A method for producing a composition comprising nemolizumab, comprising the step of purifying a solution containing nemolizumab by affinity chromatography, ion exchange chromatography, hydrophilic interaction chromatography (HILIC), hydrophobic interaction chromatography (HIC), charge-based separation, size exclusion chromatography (SEC), gel permeation chromatography (GPC), or a combination thereof.
[1.4.3] The method of [1.4.2], wherein the purifying step comprises purifying a composition comprising nemolizumab obtained from a nemolizumab-producing cell by affinity chromatography.
[1.4.4] The method of [1.4.3], wherein the hold time of the nemolizumab-containing solution after the purification by affinity chromatography is 72 hours or less, and the content of the antibody variant of any one of [1]-[7] in the composition is lower than when the hold time is longer.
[1.4.5] The method of [1.4.4], wherein the hold time of the nemolizumab-containing solution after the purification by affinity chromatography is about 24 hours or less.
[1.4.6] The method of any one of [1.4.1]-[1.4.5], wherein the antibody variant of any one of [1]-[7] is used as a reference material (standard).
[1.5.1.1] A method for reducing the content of the antibody variant of any one of [1]-[7], comprising the step of purifying an antibody-containing composition obtained from a cell producing an antibody having a variable region comprising the amino acid sequence DGYDDGPYTLET (SEQ ID NO: 3) by affinity chromatography, wherein the content of the antibody variant of any one of [1]-[7] in the composition is lower than when the method does not comprise the purifying step.
[1.5.1.2] The method of [1.5.1.1], wherein the hold time of the nemolizumab-containing solution after the purification by affinity chromatography is 48 hours or less, and the content of the antibody variant of any one of [1]-[7] in the composition is lower than when the hold time is longer.
[1.5.1.3] The method of [1.5.1.2], wherein the hold time of the nemolizumab-containing solution after the purification by affinity chromatography is about 24 hours or less.
[1.5.1.4] The method of any one of [1.5.1.1]-[1.5.1.3], wherein the antibody variant of any one of [1]-[7] is used as a reference material (standard).
[1.6.1.1] A composition comprising the antibody variant of any one of [1]-[7], wherein the antibody variant has a shorter retention time than nemolizumab when separated using anion exchange chromatography, and comprises more isoaspartic acid residues than nemolizumab.
[1.6.1.2] The composition of [1.6.1.1], wherein the content of the antibody variant is 40.3 area% or less, 30.4 area% or less, or 19.3 area% or less, and wherein the composition comprises nemolizumab.
[1.6.2.1] A composition comprising the antibody variant of any one of [1]-[7], wherein the antibody variant has a shorter retention time than nemolizumab when separated using anion exchange chromatography, and comprises more succinimide residues than nemolizumab.
[1.6.2.2] The composition of [1.6.2.1], wherein the content of the antibody variant is 29.1 area% or less, 21.6 area% or less, or 13.8 area% or less, and wherein the composition comprises nemolizumab.
[2.1.1.1] An antibody variant of nemolizumab, which has a smaller molecular weight than nemolizumab composed of two heavy chains and two light chains.
[2.1.1.2] An antibody variant of nemolizumab, which has a structure formed by two heavy chains of nemolizumab which are linked with each other via cysteines at EU numbering position 224 in the heavy chains.
[2.1.1.3] An antibody variant of nemolizumab, which has a structure formed by two light chains of nemolizumab which are linked with each other via cysteines at EU numbering position 214 in the light chains.
[2.1.1.4] An antibody variant of nemolizumab, which has a structure formed by one light chain and one heavy chain of nemolizumab, wherein a cysteine at EU numbering position 214 in the light chain is linked with a cysteine at EU numbering position 224 in the heavy chain, and cysteines at EU numbering positions 227 and 230 in the heavy chain are linked within the single heavy chain.
[2.1.1.5] An antibody variant of nemolizumab, which has a structure formed by one light chain, wherein a cysteine at EU numbering position 214 in the light chain of nemolizumab is linked with a free cysteine.
[2.1.1.6] An antibody variant of nemolizumab, which has a structure formed by two heavy chains and one light chain of nemolizumab, wherein the two heavy chains are linked with each other via cysteines at two positions: EU numbering positions 227 and 230; a cysteine at EU numbering position 224 in the first heavy chain is linked with a cysteine at EU numbering position 214 in the light chain, and a cysteine at EU numbering position 224 in the second heavy chain is linked with a free cysteine.
[2.1.2] A pharmaceutical composition comprising the antibody variant of any one of [2.1.1.1]-[2.1.1.6], wherein the ratio of the antibody variant in the total antibody molecules in the pharmaceutical composition is 11.0 CPA% or less.
[2.2.1] An antibody variant of nemolizumab, wherein asparagine at Kabat numbering position 55 in a heavy chain of nemolizumab is deamidated.
[2.2.2] A pharmaceutical composition comprising the antibody variant of [2.2.1], wherein the ratio of a deamidation-containing peptide derived from the antibody variant after enzymatic digestion of the pharmaceutical composition to the sum of the deamidation-containing peptide and the corresponding unmodified peptide is 2.0% or less.

### Effects of the Invention

The present inventors successfully identified product variants (antibody variants) with significantly lower biological activity than nemolizumab. Such antibody variants are useful as reference materials (standards) for detecting/analyzing the variants in quality assessment of nemolizumab-containing pharmaceutical compositions.

The present inventors also discovered a method for controlling (reducing) the generation of such antibody variants, namely, aspartic acid isomerization variants, and a method for measuring the content of such variants (variant detection/analysis method). These methods provide higher throughput than conventional methods, and are important techniques in quality improvement and quality management of nemolizumab. Furthermore, nemolizumab-containing pharmaceutical compositions of the present invention with a low content of such antibody variants are useful as a means for treating and/or preventing atopic dermatitis, dialysis pruritus, and other pruritus.

### [Brief Description of Drawings]

Fig. 1A is a chromatogram showing the result of separating a nemolizumab-containing solution by anion exchange high performance liquid chromatography under the conditions described in Example 1. TSK DEAE NPR (inner diameter: 4.6 mm, length: 35 mm, particle diameter 2.5 µm) was used as a column, and separation was performed under Salt Concentration Adjustment Condition 1 shown in Table 1.
Fig. 1B is a chromatogram showing the result of separating a nemolizumab-containing solution by anion exchange high performance liquid chromatography under the conditions described in Example 1. TSK DEAE NPR (inner diameter: 7.5 mm, length: 75 mm, particle diameter 2.5 µm) was used as a column, and separation was performed under Salt Concentration Adjustment Condition 2 shown in Table 2.
Fig. 2 is a schematic diagram showing the structure of the HH dimer and LL dimer, which are variants of nemolizumab.
Fig. 3 is a set of scatter plots with the X-axis representing the peptide-basis content of isoAsp forms (upper) or Asu forms (lower) contained in samples of HCCF or affinity pool held for various periods of time at 23°C, and the Y-axis representing the area ratio of Pre-Region 1 (upper) or Pre-Region 2 (lower) of AE-HPLC.
Fig. 4 is a schematic diagram showing the structure of the HL form, a variant of nemolizumab.
Fig. 5 is a set of schematic diagrams showing the structure of the cysteinylated HHL form (left) and cysteinylated light chain (right), which are variants of nemolizumab.

### [Description of Embodiments]

One embodiment of the present invention relates to antibody variants with reduced biological activity (e.g., activity of inhibiting IL-31 signaling by binding to an IL-31 receptor). In the present inventors' analysis of the drug substance of nemolizumab, these antibody variants were identified as aspartic acid isomerization variants (variants in which at least one aspartic acid residue in nemolizumab has been altered to a succinimide residue (Asu forms) or altered to an isoaspartic acid residue (isoAsp forms). Herein, an "antibody variant" may also be referred to as a product variant, or an isomerized form, an isomerization variant, or an isomer, of the original antibody.

Nemolizumab is a humanized IgG2 antibody that shows inhibitory activity on IL-31 signaling by binding to the IL-31 receptor, and is composed of heavy and light chains recognizing the IL-31 receptor. Specifically, nemolizumab comprises an H chain variable region comprising CDR1 set forth in SEQ ID NO: 1, CDR2 set forth in SEQ ID NO: 2, and CDR3 set forth in SEQ ID NO: 3, and an L chain variable region comprising CDR1 set forth in SEQ ID NO: 4, CDR2 set forth in SEQ ID NO: 5, and CDR3 set forth in SEQ ID NO: 6; More specifically, nemolizumab comprises an H chain variable region set forth in SEQ ID NO: 7 and an L chain variable region set forth in SEQ ID NO: 8. Even more specifically, nemolizumab comprises an H chain set forth in SEQ ID NO: 9 and an L chain set forth in SEQ ID NO: 10. Herein, nemolizumab is mentioned separately from its variants, and therefore the term "nemolizumab" shall not include variants of nemolizumab (e.g., aspartic acid isomerization variants).

Known methods for defining CDRs include the method according to Kabat et al. (Sequences of Proteins of Immunological Interest, 5th Ed (1991), Bethesda, MD), the method according to Chothia et al. (Science (1986) 233, 755-758), and the method based on antigen-antibody contact regions (J Mol Biol (1996) 262, 732-745). Specifically, each of the methods defines CDRs as follows:

| CDR | Kabat | Chothia | Contact |
|---|---|---|---|
| L1 | L24-L34 | L24-L34 | L30-L36 |
| L2 | L50-L56 | L50-L56 | L46-L55 |
| L3 | L89-L97 | L89-L97 | L89-L96 |
| H1 | H31-H35B | H26-H32/34 | H30-H35B (Kabat numbering) |
| H1 | H31-H35 | H26-H32 | H30-H35 (Chothia numbering) |
| H2 | H50-H65 | H52-H56 | H47-H58 |
| H3 | H95-H102 | H95-H102 | H93-H101 |

In the present disclosure, "the biological activity of an antibody variant is reduced" means that the biological activity is lower, preferably statistically significantly lower, than the biological activity of an antibody used as a reference for comparison (e.g., nemolizumab). In one embodiment, the biological activity of the antibody variant of the present invention is 10% or more lower, for example, 15% or more, 20% or more, 25% or more, 30% or more, 35% or more, 40% or more, 45% or more, 50% or more, 55% or more, 59% or more, 60% or more, 65% or more, 70% or more, 75% or more, 79% or more, 80% or more, 85% or more, or 90% or more lower, than the biological activity of an antibody as a reference for comparison (e.g., nemolizumab). In one embodiment, the biological activity of the antibody variant of the present invention relative to that of an antibody used as a reference for comparison (e.g., CIM331 reference material, i.e., nemolizumab) (relative biological activity) is 90% or lower, for example, 85% or lower, 80% or lower, 75% or lower, 70% or lower, 65% or lower, 60% or lower, 55% or lower, 50% or lower, 45% or lower, 41% or lower, 40% or lower, 35% or lower, 30% or lower, 25% or lower, 21% or lower, 20% or lower, 15% or lower, 10% or lower, or 5% or lower.

In one embodiment, the biological activity of nemolizumab or an antibody variant thereof is neutralization activity against the IL-31 receptor. In one embodiment, the neutralization activity against the IL-31 receptor is the activity of inhibiting binding between IL-31 and the IL-31 receptor. In one embodiment, the neutralization activity against the IL-31 receptor is inhibitory activity on IL-31 signaling by binding to the IL-31 receptor (e.g., the activity of suppressing IL-31-dependent cell proliferation). As described in WO2010/064697, nemolizumab (CIM331) has neutralization activity against human IL-31RA and cynomolgus IL-31RA. Such neutralization activity can be assessed, for example, using the method described in WO2010/064697.

In one method, whether nemolizumab or an antibody variant thereof inhibits IL-31 signaling can be verified by examining whether nemolizumab or the antibody variant thereof inhibits binding of IL-31 to the IL-31 receptor. Examples of methods for making such a determination include an assay using ELISA or flow cytometry and an assay using surface plasmon resonance. With ELISA, for example, whether nemolizumab or the antibody variant thereof inhibits the binding of IL-31 to the IL-31 receptor can be evaluated by immobilizing the IL-31 receptor (or IL-31RA) protein onto a plate, preparing a system for detecting the amount of IL-31 protein that binds thereto through the use of a secondary antibody such as an enzyme-labeled anti-IL-31 antibody, and determining whether or not the addition of nemolizumab or the antibody variant thereof reduces the amount of detected IL-31 protein.

In an alternative method, whether nemolizumab or an antibody variant thereof inhibits IL-31 signaling can be verified by examining whether the bioactivity induced by the action of IL-31 on cells is inhibited by nemolizumab or the antibody variant thereof. The bioactivity is not particularly limited as long as it can be quantitatively or qualitatively determined using any method, and examples of such bioactivities include cell proliferative activity, protein phosphorylation activity, and gene/protein expression-inducing activity. For example, whether nemolizumab or the antibody variant thereof inhibits IL-31 signaling can be evaluated by preparing cells that express the IL-31 receptor on the surface, and whose proliferative activity is induced in response to external IL-31 stimulation, and determining whether or not the addition of nemolizumab or the antibody variant thereof reduces the IL-31-induced cell proliferative activity. As such cells, naturally occurring cells inherently expressing the IL-31 receptor may be used, or recombinant cells artificially synthesized to express the IL-31 receptor may be used. A suitable example of recombinant cells includes Ba/F3 cells expressing the IL-31 receptor. As a further alternative, the method described in the document of Dillon et al. (Nat Immunol (2004) 5, 752-760) may be used.

In the present disclosure, the degree of inhibition of IL-31 signaling by nemolizumab or an antibody variant thereof is not limited, but may be, for example, at least 10% or more, preferably 20% or more, 30% or more, 40% or more, 50% or more, 60% or more, 70% or more, and 80% or more, 90% or more, 95% or more, or 98% or more.

The term "antibody" is used in the broadest sense, and includes monoclonal antibodies, polyclonal antibodies, dimers, multimers, multispecific antibodies (such as bispecific antibodies), antibody derivatives, and modified antibodies (Miller K et al. J Immunol. 2003, 170(9), 4854-61) so long as they show a desired biological activity (e.g., binding activity to an antigen). The antibodies may be mouse antibodies, human antibodies, humanized antibodies, and chimeric antibodies, or may be those derived from another species, or artificially synthesized antibodies. The antibodies disclosed herein can be of any type (for example, IgG, IgE, IgM, IgD, and IgA), class (for example, IgGl, IgG2, IgG3, IgG4, IgAl and IgA2) or subclass of immunoglobulin molecules. The immunoglobulins can be derived from any species (for example, human, mouse, or rabbit). The terms "antibody", "immune globulin" and "immunoglobulin" are used interchangeably in a broad sense.

Further, an antibody may not only be an antibody molecule composed of two heavy chains and two light chains, but also an antibody fragment with a smaller molecular weight. For example, the definition of antibody herein also includes molecules having a structure formed of two heavy chains, a structure formed of two light chains, a structure formed of one light chain and one heavy chain, a structure formed of one light chain linked to free cysteine, and a structure formed of two heavy chains and one light chain. In light of this broad definition, the antibody variants of the present invention are also included in "antibodies". Thus, "(all) antibody molecules in a pharmaceutical composition" herein may include both an antibody and variants thereof.

Recombinant antibodies produced by using genetic engineering techniques can be used as the antibodies. A recombinant antibody can be obtained by cloning a DNA encoding the antibody from hybridomas or antibody-producing cells such as sensitized lymphocytes that produce antibodies; inserting this into a vector; and then introducing it into hosts (host cells) to produce the antibody.

In the present invention, antibodies can be produced by methods known to those skilled in the art. Specifically, a DNA encoding the antibody of interest is inserted into an expression vector. The insertion into the expression vector is carried out such that the expression will take place under the control of expression regulatory regions such as an enhancer and a promoter. Next, host cells are transformed using this expression vector to express the antibody. Appropriate combinations of a host and an expression vector can be used in this case.

The antibodies thus obtained can be isolated from the inside of host cells or the outside of the cells (medium, etc.), and purified. The antibodies can be separated and purified by methods ordinarily used for separating and purifying antibodies, and the methods are not limited in any way. For separation and purification of the antibodies and the antibody variants in the present invention, for example, the antibodies can be separated and purified by appropriately selecting and combining column chromatography, filtration, ultrafiltration, salting-out, solvent precipitation, solvent extraction, distillation, immunoprecipitation, SDS-polyacrylamide gel electrophoresis, isoelectric focusing, dialysis, recrystallization, and such. For example, in the separation and purification using a chromatography column, various types of matrix including an anion exchange matrix, cation exchange matrix, anti-human IgG affinity matrix, and protein L matrix can be used.

Without being bound by a particular theory, the antibody variant of the present invention may be a product variant of an antibody molecule that has a variable region containing the amino acid sequence DGYDDGPYTLET (SEQ ID NO: 3) (e.g., nemolizumab), generated in the manufacturing process (e.g., purification step) of the antibody molecule. In one aspect, the antibody variant of the present invention has at least one (e.g., one, two, or three) amino acid residue altered relative to an antibody molecule that has a variable region containing the amino acid sequence DGYDDGPYTLET (SEQ ID NO: 3) (e.g., nemolizumab). In one embodiment, the antibody variant of the present invention has one or more, three or less amino acid residues, specifically, one, two, or three amino acid residues altered relative to an antibody molecule that has a variable region containing the amino acid sequence DGYDDGPYTLET (SEQ ID NO: 3) (e.g., nemolizumab). In a specific embodiment, the antibody variant of the present invention has at least one (e.g., one, two, or three) amino acid residue altered in the amino acid sequence DGYDDGPYTLET (SEQ ID NO: 3) relative to an antibody molecule that has a variable region containing the amino acid sequence DGYDDGPYTLET (SEQ ID NO: 3) (e.g., nemolizumab). In a specific embodiment, the antibody variant of the present invention has one or more, three or less amino acid residues, specifically, one, two, or three amino acid residues altered in the amino acid sequence DGYDDGPYTLET (SEQ ID NO: 3) relative to an antibody molecule that has a variable region containing the amino acid sequence DGYDDGPYTLET (SEQ ID NO: 3) (e.g., nemolizumab).

In another aspect, the antibody variant of the present invention has a different combination of SS bonds (bonds between cysteine residues or bonds between a cysteine residue and free cysteine) relative to an antibody molecule that has a variable region containing the amino acid sequence DGYDDGPYTLET (SEQ ID NO: 3) (e.g., nemolizumab). In one embodiment of this aspect, the antibody variant of the present invention has the same amino acid sequence as, but a different combination of SS bonds (bonds between cysteine residues or bonds between a cysteine residue and free cysteine) and a different molecular weight relative to, an antibody molecule that has a variable region containing the amino acid sequence DGYDDGPYTLET (SEQ ID NO: 3) (e.g., nemolizumab). In another embodiment, the antibody variant of the present invention has a different amino acid sequence, a different combination of SS bonds (bonds between cysteine residues or bonds between a cysteine residue and free cysteine), and a different molecular weight, relative to an antibody molecule that has a variable region containing the amino acid sequence DGYDDGPYTLET (SEQ ID NO: 3) (e.g., nemolizumab).

In one aspect, the present invention relates to antibody variants having the following features:
- one or more aspartic acid (Asp) residues in the heavy chain CDRs of an antibody having a variable region containing the amino acid sequence DGYDDGPYTLET (SEQ ID NO: 3) (e.g., nemolizumab) are changed to succinimide (Asu) residues or isoaspartic acid (isoAsp) residues;
- the biological activity (activity of inhibiting IL-31 signaling by binding to the IL-31 receptor) is extremely low as compared to the above-mentioned antibody (e.g., nemolizumab).
- the amount of generation increases in a manner dependent on the hold time of an antibody-containing solution after purification of the above-mentioned antibody (e.g., nemolizumab) by affinity chromatography and before the next step.

Antibody variants with these features may be herein referred to as aspartic acid isomerization variants or aspartic acid isomerization forms. In a specific embodiment, the above-mentioned antibody is nemolizumab, and the above-mentioned amino acid sequence is the sequence of heavy chain CDR3 of nemolizumab.

In one embodiment, the aspartic acid isomerization variant of the present invention is a variant of an antibody having a variable region containing the amino acid sequence DGYDDGPYTLET (SEQ ID NO: 3), wherein:
(a) the amino acid residue D (Asp) at position 1 from the N-terminus of the above sequence, or
(b) the amino acid residue D (Asp) at position 4 from the N-terminus of the above sequence,
   or
(c) the amino acid residue D (Asp) at position 5 from the N-terminus of the above sequence is a succinimide residue (which may be herein referred to as a succinimide form or an Asu form), or an isoaspartic acid residue (which may be herein referred to as an isoaspartic acid form or an isoAsp form). The position of the succinimide residue in the succinimide form is preferably position 1 or 5 from the N-terminus of the above-mentioned sequence (SEQ ID NO: 3), and particularly preferably position 5 from the N-terminus of the above-mentioned sequence.

In a specific embodiment, the aspartic acid isomerization variant of the present invention is a variant of an antibody having a variable region containing the amino acid sequence DGYDDGPYTLET (SEQ ID NO: 3), wherein:
(a2) the amino acid residue D (Asp) at positions 1 and 5 from the N-terminus of the above sequence,
   or
(b2) the amino acid residue D (Asp) at positions 4 and 5 from the N-terminus of the above sequence,
   or
(c2) the amino acid residue D (Asp) at positions 1 and 4 from the N-terminus of the above sequence,
is a succinimide (Asu) residue or an isoaspartic acid (isoAsp) residue. Examples of such antibody variants include antibody variants which comprise:
   an isoAsp residue at position 1 and an Asu residue at position 5;
   an Asu residue at position 1 and isoAsp at position 5;
   Asu residues at positions 1 and 5;
   isoAsp residues at positions 1 and 5;
   an isoAsp residue at position 4 and an Asu residue at position 5;
   an Asu residue at position 4 and an isoAsp residue at position 5;
   Asu residues at positions 4 and 5;
   isoAsp residues at positions 4 and 5;
   an isoAsp residue at position 1 and an Asu residue at position 4;
   an Asu residue at position 1 and an isoAsp residue at position 4;
   Asu residues at positions 1 and 4; or
   isoAsp residues at positions 1 and 4,
   from the N-terminus of the above amino acid sequence (SEQ ID NO: 3), and preferably include antibody variants which comprise:
      isoAsp residues at positions 1 and 4;
      isoAsp residues at positions 1 and 5;
      isoAsp residues at positions 4 and 5;
      an isoAsp residue at position 1 and an Asu residue at position 5; or
      an isoAsp residue at position 4 and an Asu residue at position 5;
from the N-terminus of the above amino acid sequence (SEQ ID NO: 3).

In a specific embodiment, the above-mentioned antibody is nemolizumab, and the above-mentioned amino acid sequence is the sequence of heavy chain CDR3 of nemolizumab.

In another aspect, the present invention also relates to methods for detecting, and methods for analyzing, aspartic acid isomerization variants. In one embodiment, the method of the present invention comprises the step of separating a sample comprising an antibody having a variable region comprising the amino acid sequence of DGYDDGPYTLET (SEQ ID NO: 3) or the antibody that has been enzymatically treated, by affinity chromatography, ion exchange chromatography, normal-phase chromatography, reverse-phase chromatography, hydrophilic interaction chromatography (HILIC), hydrophobic interaction chromatography (HIC), charge-based separation, size exclusion chromatography (SEC), gel permeation chromatography (GPC), or a combination thereof. In a specific embodiment, the method of the present invention comprises, prior to the separation step, the step of enzymatically treating a sample comprising an antibody. Enzymes to treat a sample comprising an antibody with include, but are not limited to, trypsin and Lys-C. In a specific embodiment, the method of the present invention comprises, following the step of separating a sample comprising the antibody that has been enzymatically treated, the step of identifying and/or quantifying a peptide by mass spectrometry and/or ultraviolet absorption spectroscopy.

In the method of the present invention, detection and analysis can be performed using as an index the presence/absence of a structural change in aspartic acid in a CDR having the amino acid sequence of SEQ ID NO: 3. Such a structural change can be detected, for example, using as an index a shift in the molecular weight or retention time in LCMS analysis due to the structural change. Alternatively, the structural change can be detected using a difference in the degree of separation with ion exchange chromatography. For example, when anion exchange chromatography is used for separation, the aspartic acid isomerization variants of the present invention are separated in a more basic region (region with a shorter retention time) than the main peak of the main component, namely nemolizumab.

In one embodiment of the method of the present invention, the ion exchange chromatography is anion exchange chromatography. In one embodiment, anion exchange chromatography is characterized by using a column with an inner diameter of 4.6-7.5 mm, a length of 35-150 mm, and a particle diameter of 2.5-10 µm at a column temperature of 25-50°C and performing elution with a mobile phase of pH6.0-10.0. In a specific embodiment, anion exchange chromatography is characterized by using a column with an inner diameter of 7.5 mm, a length of 75 mm, and a particle diameter of 2.5 µm at a column temperature of 40°C and performing elution with a mobile phase of pH7.5 containing 250 mmol/L sodium chloride.

Moreover, in one embodiment, the method of the present invention comprises the step of performing one or more analyses selected from the group consisting of quantitative analysis, qualitative analysis, and structural analysis, using, as a standard:
(i) an aspartic acid isomerization variant of the present invention,
(ii) a peptide containing an isoAsp or Asu residue obtained by enzymatic digestion of the variant, or
(iii) a peptide containing the amino acid sequence of DGYDDGPYTLET (SEQ ID NO: 3) in which one, two, or three of the aspartic acid (Asp, D) residues at positions 1, 4, and 5 from the N-terminus of the sequence have been altered to a succinimide (Asu) residue or an isoaspartic acid (isoAsp) residue. Examples of the peptide of (iii) include peptides in which one of positions 1, 4, and 5 from the N-terminus of the amino acid sequence of SEQ ID NO: 3 has been altered to an Asu residue or an isoAsp residue. Other examples of the peptide of (iii) include peptides in which two of positions 1, 4, and 5 from the N-terminus of the amino acid sequence of SEQ ID NO: 3 have been altered to an Asu residue or an isoAsp residue, and which comprise:
   an isoAsp residue at position 1 and an Asu residue at position 5;
   an Asu residue at position 1 and an isoAsp residue at position 5;
   Asu residues at positions 1 and 5;
   isoAsp residues at positions 1 and 5;
   an isoAsp residue at position 4 and an Asu residue at position 5;
   an Asu residue at position 4 and an isoAsp residue at position 5;
   Asu residues at positions 4 and 5;
   isoAsp residues at positions 4 and 5;
   an isoAsp residue at position 1 and an Asu residue at position 4;
   an Asu residue at position 1 and an isoAsp residue at position 4;
   Asu residues at positions 1 and 4; or
   isoAsp residues at positions 1 and 4;
and preferably include peptides which comprise:
isoAsp residues at positions 1 and 4;
isoAsp residues at positions 1 and 5;
isoAsp residues at positions 4 and 5;
an isoAsp residue at position 1 and an Asu residue at position 5; or
an isoAsp residue at position 4 and an Asu residue at position 5.

In another aspect, the present invention enables production and quality control of a pharmaceutical composition containing nemolizumab by performing one or a combination of the detection methods and analysis methods as mentioned above. Accordingly, the present invention relates to methods for quality control of a pharmaceutical composition containing nemolizumab, comprising performing a detection method and an analysis method as mentioned above, or combining these methods. The present invention also relates to methods for producing a pharmaceutical composition containing nemolizumab, comprising performing one or more of such detection methods, analysis methods, and quality control methods.

In the production method of the present invention, a nemolizumab-containing pharmaceutical composition with a low content of aspartic acid isomerization variants can be produced by purifying a solution containing nemolizumab by affinity chromatography, ion exchange chromatography, hydrophilic interaction chromatography (HILIC), hydrophobic interaction chromatography (HIC), charge-based separation, size exclusion chromatography (SEC), gel permeation chromatography (GPC), or a combination thereof. Such a nemolizumab-containing pharmaceutical composition with a low content of aspartic acid isomerization variants is useful as a means for treating and/or preventing diseases on which nemolizumab exerts therapeutic and/or preventive effects, such as atopic dermatitis, dialysis pruritus, and other pruritus.

In one embodiment, the production method of the present invention comprises culturing nemolizumab-producing cells (e.g., CHO cells) into which a gene encoding nemolizumab has been introduced. This culturing step can be performed under conditions that are known to persons skilled in the art as appropriate for culturing nemolizumab-producing cells (e.g., CHO cells). For example, commercially available basal media (basal media for animal cell culture) can be used.

In one embodiment, the production method of the present invention comprises centrifuging and filtering culture fluid of nemolizumab-producing cells to obtain a nemolizumab-containing solution (Harvest Cell Culture Fluid: HCCF). Purification of nemolizumab from the obtained nemolizumab-containing solution can be carried out by a combination of common column chromatography methods, such as affinity chromatography, ion exchange chromatography, and hydrophobic chromatography. In a specific embodiment, the production method of the present invention comprises purifying a nemolizumab-containing solution obtained from nemolizumab-producing cells by affinity chromatography (e.g., protein A affinity chromatography). The hold time of the antibody-containing solution after the affinity chromatography purification and before the next step is not particularly limited, but shortening this time can reduce the content of aspartic acid isomerization variants. Therefore, in order to maintain the biological activity of the antibody and antibody variants in a nemolizumab-containing pharmaceutical composition obtained by the production method of the present invention at acceptable levels, it is preferred that the hold time of the antibody-containing solution after the affinity chromatography purification and before the next step be within a prescribed range (e.g., within about 138 hours, within about 72 hours, within about 48 hours, or within about 24 hours).

In another aspect, the present invention relates to pharmaceutical compositions comprising an antibody having a variable region comprising the amino acid sequence DGYDDGPYTLET (SEQ ID NO: 3) (e.g., nemolizumab) and variants thereof (e.g., aspartic acid isomerization variants), in which the ratio of the variants to all antibody molecules in the pharmaceutical composition (including the aforementioned antibody and variants thereof) is reduced. Such pharmaceutical compositions of the present invention in which the ratio of the variants is reduced can be suitably used in treatment and/or prevention of atopic dermatitis, dialysis pruritus, and other pruritus. In one embodiment, the pharmaceutical composition of the present invention is a pharmaceutical composition for use in either or both of treatment and prevention of at least one of atopic dermatitis, dialysis pruritus, and other pruritus. The present invention also relates to methods for treating or preventing at least one of atopic dermatitis, dialysis pruritus, and other pruritus, comprising administering the pharmaceutical composition of the present invention.

In one embodiment, the pharmaceutical composition of the present invention is obtained by a purification step including purification by affinity chromatography (e.g., protein A affinity chromatography). As described above, for example, by setting the hold time of the antibody-containing solution after purification of the antibody solution containing the aforementioned antibody (e.g., nemolizumab) by protein A affinity chromatography and before the next step (e.g., the hold time at 18 to 28°C or 18 to 23°C, or preferably the hold time at room temperature, 18°C, or 23°C) to a short time, the ratio of aspartic acid isomerization variants to all antibody molecules in the resulting composition can be reduced.

The ratio of aspartic acid isomerization variants in the antibody molecules in the pharmaceutical composition of the present invention can be evaluated by various methods including the above-mentioned methods for detecting/analyzing aspartic acid isomerization variants. Known methods for analyzing proteins such as antibodies include, for example, peptide mapping methods, in which a protein is degraded into peptide fragments by enzymatic digestion and such, and they are separated by various chromatography techniques and subjected to mass spectrometry and ultraviolet absorption spectroscopy to obtain the amino acid sequence and posttranslational modification information of the protein. As described in the Examples herein, the structure and content of aspartic acid isomerization variants of the present invention can be analyzed by peptide mapping methods.

In one embodiment, the ratio of aspartic acid isomerization variants in the antibody molecules in the pharmaceutical composition of the present invention, can be represented by the ratio of succinimide residue-containing peptides and isoaspartic acid residue-containing peptides derived from the aspartic acid isomerization variants obtained by enzymatic digestion of the pharmaceutical composition of the present invention, to the sum of these variant-derived peptides and the corresponding unmodified peptides (unmodified peptides which have the same amino acid sequence as the variant-derived peptides except that the sites modified to succinimide or isoaspartic acid residue are aspartic acid residues) (this may be herein referred to as the ratio of aspartic acid isomerization variants on peptide basis). Digestion enzymes that can be used include, for example, trypsin and Lys-C. The ratio of isoaspartic acid forms (isoAsp forms) on peptide basis in the pharmaceutical composition of the present invention (the ratio in comparison to the aspartic acid form on peptide basis) is, for example, 25% or lower, 20% or lower, 15% or lower, or 9.4% or lower. The ratio of succinimide forms (Asu forms) on peptide basis in the pharmaceutical composition of the present invention (the ratio in comparison to the aspartic acid form on peptide basis) is, for example, 25% or lower, 20% or lower, 15% or lower, or 9.8% or lower.

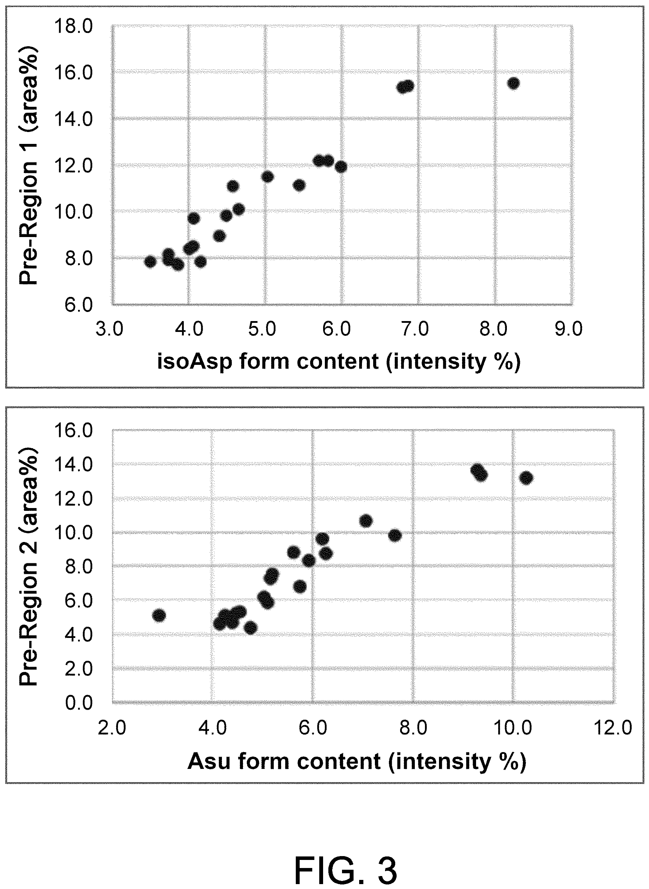

As described in Example 6 herein, the ratio of succinimide residue-containing peptides and isoaspartic acid residue-containing peptides derived from the aspartic acid isomerization variants obtained after enzymatic digestion, to the sum of these variant-derived peptides and the corresponding unmodified peptides (unmodified peptides which have the same amino acid sequence as the variant-derived peptides except that the sites modified to succinimide or isoaspartic acid residue are aspartic acid residues) (the ratio of aspartic acid isomerization variants on peptide basis), correlates with the ratio of the peak area of the elution peaks of aspartic acid isomerization variants in anion exchange chromatography (AE-HPLC) to the total peak area (peak area ratio) (Fig. 3). Therefore, the ratio of aspartic acid isomerization variants in the antibody molecules in the pharmaceutical composition of the present invention, can be represented by the ratio of the peak area of all elution peaks of succinimide forms (Asu forms) and isoaspartic acid forms (isoAsp forms) together to the total peak area (peak area ratio) in anion exchange chromatography (AE-HPLC) analysis of the pharmaceutical composition of the present invention. This evaluation method provides higher throughput than peptide mapping-based detection/analysis, and is advantageous in quality control of nemolizumab.

In one embodiment, the elution fractions of isoaspartic acid forms (isoAsp forms) and succinimide forms (Asu forms) in anion exchange chromatography (AE-HPLC) analysis of the pharmaceutical composition of the present invention are in a more basic region (a region with a shorter retention time) than the main peak including nemolizumab, i.e., the main component of the pharmaceutical composition of the present invention, and this may be herein referred to as Pre-Region. Of the regions that contain eight peaks included in this region (Pre-Region), the region containing three peaks adjacent to the main peak including nemolizumab (which may be herein referred to as Pre-Region 1) is where the isoaspartic acid forms (isoAsp forms) are predominantly eluted, and the region containing the other five peaks (which may be herein referred to as Pre-Region 2) is where the succinimide forms (Asu forms) are predominantly eluted. Therefore, when evaluated by the analysis method of the present invention using anion exchange chromatography (AE-HPLC), the ratios of isoaspartic acid forms (isoAsp forms) and succinimide forms (Asu forms) to all antibody molecules in the pharmaceutical composition of the present invention can be represented by the peak area ratios of Pre-Region 1 and Pre-Region 2, respectively.

In one embodiment, when evaluated by the analysis method of the present invention using anion exchange chromatography (AE-HPLC), the ratio of isoaspartic acid forms (isoAsp forms) to all antibody molecules in the pharmaceutical composition of the present invention (the area ratio of Pre-Region 1) is, for example, 50.2 area% or less, 40.3 area% or less, 30.4 area% or less, or 19.3 area% or less. In one embodiment, when evaluated by the analysis method of the present invention using anion exchange chromatography (AE-HPLC), the ratio of succinimide forms (Asu forms) to all antibody molecules in the pharmaceutical composition of the present invention (the area ratio of Pre-Region 2) is, for example, 36.6 area% or less, 29.1 area% or less, 21.6 area% or less, or 13.8 area% or less.

Moreover, from the ratio of aspartic acid isomerization variants on peptide basis, the theoretical maximum ratio of aspartic acid isomerization variants on antibody basis can be calculated based on a binomial distribution as described in Example 6 herein.

The ratio of isoaspartic acid forms (isoAsp forms), to the sum of the isoAsp forms and the unmodified antibody (ratio on antibody basis) in the antibody molecules in the pharmaceutical composition of the present invention, based on the calculation from the ratio on peptide basis of aspartic acid isomerization variants is, for example, 43.8% or less, 36.0% or less, 27.8% or less, or 17.9% or less. Further, the ratio on antibody basis of isoAsp forms in the antibody molecules in the pharmaceutical composition of the present invention in which the two heavy chains contain isoAsp residues is, for example, 6.3% or less, 4.0% or less, 2.3% or less, or 0.9% or less. The ratio on antibody basis of isoAsp forms in the antibody molecules in the pharmaceutical composition of the present invention in which one of the two heavy chains contains an isoAsp residue(s) is, for example, 37.5% or less, 32.0% or less, 25.5% or less, or 17.0% or less.

Similarly, the ratio of succinimide forms (Asu forms), to the sum of the Asu forms and the unmodified antibody in the antibody molecules in the pharmaceutical composition of the present invention (ratio on antibody basis) is, for example, 43.8% or less, 36.0% or less, 27.8% or less, or 18.7% or less. Further, the ratio of Asu forms in which the two heavy chains contain isoAsp residues to the sum of the Asu forms and the unmodified antibody in the antibody molecules in the pharmaceutical composition of the present invention is, for example, 6.3% or less, 4.0% or less, 2.3% or less, or 1.0% or less. The ratio of Asu forms in which one of the two heavy chains contains an isoAsp residue(s) to the sum of the Asu forms and the unmodified antibody in the antibody molecules in the pharmaceutical composition of the present invention is, for example, 37.5% or less, 32.0% or less, 25.5% or less, or 17.7% or less.

In another aspect, the present invention relates to methods for producing a pharmaceutical composition in which the content of aspartic acid isomerization variants is reduced, and methods for suppressing the generation of aspartic acid isomerization variants. The amount of aspartic acid isomerization variants generated can be reduced by setting the hold time of an antibody-containing solution that contains an antibody with a variable region containing the amino acid sequence DGYDDGPYTLET (SEQ ID NO: 3) (e.g., nemolizumab) after purifying the antibody-containing solution by affinity chromatography (e.g., protein A affinity chromatography) and before the next step to a short time. Therefore, the above method comprises purifying an antibody-containing composition obtained from cells producing an antibody having a variable region comprising the amino acid sequence of DGYDDGPYTLET (SEQ ID NO: 3) (e.g., nemolizumab) by affinity chromatography (e.g., protein A affinity chromatography), and is characterized in that the hold time of the antibody-containing solution after the affinity chromatography purification and before the next step is short. In one embodiment, the hold time of the antibody-containing solution after affinity chromatography purification and before the next step (for example, the hold time at 18-28°C or 18-23°C, or preferably at room temperature, 18°C, or 23°C) is about 138 hours or shorter, for example, about 72 hours or shorter, about 48 hours or shorter, or about 24 hours or shorter, and preferably about 48 hours or shorter, and more preferably about 24 hours or shorter.

In another aspect, the present invention relates to a method for purifying an antibody-containing composition, comprising purifying an antibody-containing composition (e.g., a harvest cell culture fluid (HCCF) obtained by centrifuging and filtering the culture fluid of antibody-producing cells) by affinity chromatography (e.g., protein A affinity chromatography). In one embodiment of the above method, the antibody is an antibody having a variable region comprising the amino acid sequence of DGYDDGPYTLET (SEQ ID NO: 3). In a specific embodiment, the antibody is nemolizumab. In one embodiment of the above method, the hold time of the antibody-containing solution after affinity chromatography purification and before the next step (for example, the hold time at 18-28°C or 18-23°C, or preferably at room temperature, 18°C, or 23°C) is about 138 hours or shorter, for example, about 72 hours or shorter, about 48 hours or shorter, or about 24 hours or shorter. It is preferably about 72 hours or shorter, more preferably about 48 hours or shorter, and even more preferably about 24 hours or shorter.

In another aspect, the present invention relates to antibody variants having one of the following structures:
(1) a structure formed by two heavy chains of nemolizumab which are linked with each other via cysteines at EU numbering position 224 in the heavy chains (HH dimer);
(2) a structure formed by two light chains of nemolizumab which are linked with each other via cysteines at EU numbering position 214 in the light chains (LL dimer);
(3) a structure formed by one light chain and one heavy chain of nemolizumab, wherein a cysteine at EU numbering position 214 in the light chain is linked with a cysteine at EU numbering position 224 in the heavy chain, and cysteines at EU numbering positions 227 and 230 in the heavy chain are linked within the single heavy chain (HL form);
(4) a structure formed by one light chain, wherein a cysteine at EU numbering position 214 in the light chain of nemolizumab is linked with a free cysteine (cysteinylated light chain); or
(5) a structure formed by two heavy chains and one light chain of nemolizumab,
wherein the two heavy chains are linked with each other via cysteines at two positions: EU numbering positions 227 and 230; a cysteine at EU numbering position 224 in the first heavy chain is linked with a cysteine at EU numbering position 214 in the light chain, and a cysteine at EU numbering position 224 in the second heavy chain is linked with a free cysteine (cysteinylated HHL form).

Antibody variants having these structures are low molecular weight variants (LMWS: low molecular weight species) which have a lower molecular weight than nemolizumab which is composed of two heavy chains and two light chains. Such variants may be herein referred to as low molecular weight variants of nemolizumab. In a further aspect, the present invention relates to pharmaceutical compositions comprising nemolizumab and low molecular weight variants (LMWS) thereof, wherein the ratio of the low molecular weight variants to all antibody molecules in the pharmaceutical composition is reduced (for example, the ratio is 11.0 CPA (corrected peak area) % or less).

In another aspect, the present invention relates to antibody variants of nemolizumab in which asparagine at Kabat numbering position 55 in the heavy chain is deamidated, and pharmaceutical compositions comprising such an antibody variant. In one embodiment, the ratio of a deamidation-containing peptide, derived from the antibody variant after enzymatic digestion of the pharmaceutical composition, to the sum of the deamidation-containing peptide and the corresponding unmodified peptide is 2.0% or less.

As used herein, aspects referred to by the expression "comprising" include those referred to by the expression "essentially consisting of", and those referred to by the expression "consisting of".

Numerical values recited herein may vary within a certain range, for example, depending on the instruments or equipment, measurement conditions, and procedure used by those skilled in the art, and so long as they are within a range that allows the objective of the invention to be accomplished, they may encompass a deviation of approximately 10%, for example.

All patents and references explicitly cited herein are incorporated by reference into this specification in its entirety.

The present invention will be further illustrated by the Examples below, but it is not to be construed as being limited thereto.

### [Examples]

### [Example 1] Separation of nemolizumab-containing solution by anion exchange high performance liquid chromatography (HPLC)

### [1.1] Preparation of nemolizumab-containing solution

Nemolizumab, an anti-human IL-31RA antibody described in WO2010/064697 (also known as CIM331; H chain, SEQ ID NO: 9; L chain, SEQ ID NO: 10), was produced by a method known to persons skilled in the art in accordance with the description of the above patent document. Briefly, a nemolizumab-containing solution (drug substance) was produced by a method that comprises culturing host cells containing a vector into which a gene encoding nemolizumab was introduced, and purifying a solution containing nemolizumab obtained by the culturing step. This drug substance was used to produce a nemolizumab drug product by the method described in WO2021/100794.

### [1.2] Separation of nemolizumab-containing solution by anion exchange high performance liquid chromatography

To an aliquot of the nemolizumab-containing solution, mobile phase A was added to give 0.5 mg/mL of nemolizumab (total concentration of nemolizumab and its variants), and this was used as a sample solution. Mobile phase A was used as a blank test solution. Separation tests using anion exchange high performance liquid chromatography were performed for 20 µL (or 10 µL when TSK DEAE NPR (inner diameter: 4.6 mm, length: 35 mm, particle diameter: 2.5 µm) and Salt Concentration Gradient Condition 1 were used) of the sample solution and the blank test solution under the following conditions:

### [Test conditions]

Detector: Ultraviolet-visible absorption spectrophotometer (measured wavelength: 280 nm)
Column: TSK DEAE NPR (inner diameter: 4.6 mm, length: 35 mm, particle size: 2.5 µm), TSK DEAE NPR (inner diameter: 7.5 mm, length: 75 mm, particle diameter: 2.5 µm), or their equivalents
Guard column: TSK guard column DEAE NPR (inner diameter: 4.6 mm, length: 5 mm, particle diameter: 5.0 µm)
Column temperature: constant temperature around 40°C
Mobile phase A: 25 mmol/L Tris buffer, pH 7.5 ± 0.1
Mobile phase B: 25 mmol/L Tris buffer containing 250 mmol/L sodium chloride, pH 7.5 ± 0.1
Flow volume: 1.0 mL/min
Delivery of mobile phases: The mixing ratio of mobile phases A and B was changed as follows for a controlled concentration gradient:

**Table 1]**

| Salt Concentration Gradient Condition 1 | | | |
|---|---|---|---|
| Time (min) | 0 | 5 | 45 |
| Mobile phase A (vol%) | 60 | 60 | 0 |
| Mobile phase B (vol%) | 40 | 40 | 100 |

**Table 2**

| Salt Concentration Gradient Condition 2 | | | | | |
|---|---|---|---|---|---|
| Time (min) | 0 | 5 | 45 | 55 | 65 |
| Mobile phase A (vol%) | 60 | 60 | 30 | 0 | 0 |
| Mobile phase B (vol%) | 40 | 40 | 70 | 100 | 100 |

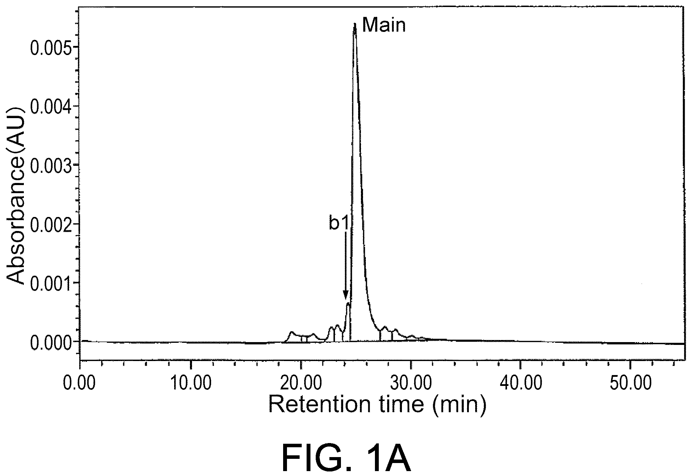

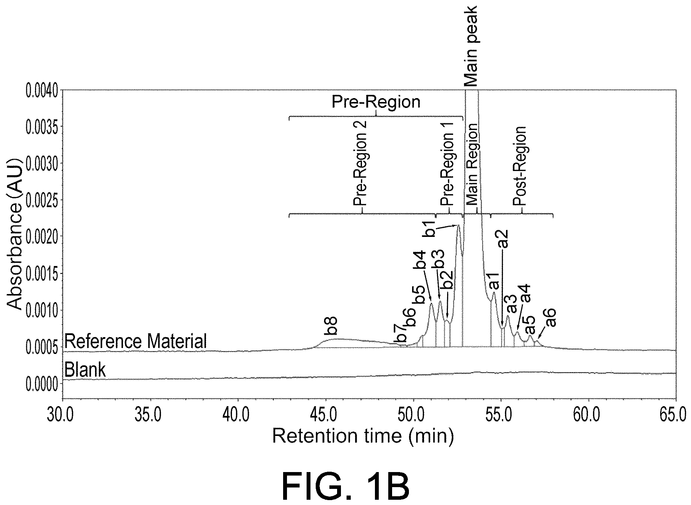

When separation was performed using TSK DEAE NPR (inner diameter: 4.6 mm, length: 35 mm, particle diameter 2.5 µm) as a column under Concentration Adjustment Condition 1 above, the chromatogram as shown in Fig. 1A was obtained. Meanwhile, when separation was performed using TSK DEAE NPR (inner diameter: 7.5 mm, length: 75 mm, particle diameter 2.5 µm) as a column under Concentration Adjustment Condition 2 above, the chromatogram as shown in Fig. 1B was obtained.

As shown in Fig. 1A-B, the region with an earlier retention time (shorter retention time) than the Main Region containing the main peak was designated as Pre-Region (the region containing fractions eluted at lower salt concentrations than the Main Region), and the region with an later retention time (longer retention time) than the Main Region was designated as Post-Region (the region containing fractions eluted at higher salt concentrations than the Main Region). Also, the peaks in the Pre-Region were designated as peaks b1 to b8 in order of proximity to the Main Region (i.e., in descending order of retention time), and the peaks in the Post Region were designated as peaks a1 to a6 in order of proximity to the Main Region (i.e., in ascending order of retention time). The Pre-Region was separated into, and designated as, Pre-Region 1 (region containing peaks b1 to b3) and Pre-Region 2 (region containing peaks b4 to b8) in descending order of retention time.

In comparison between Figs. 1A and 1B, Fig .1B showed better separation between peak b1 and the main peak. Thus, in the separation test below, the conditions of Fig. 1B (column: TSK DEAE NPR (inner diameter: 7.5 mm, length: 75 mm, particle diameter 2.5 µm); salt concentration gradient condition: Table 2) were used.

The information of nemolizumab variants contained in each peak of Pre-Region 1, Pre-Region 2, and Post-Region shown in Fig. 1B is summarized in Table 3 below. The biological activity in Table 3 is the result of evaluation as described in Example 5.

**[Table 3]**

| Region name | Peak name | Relative biological activity compared to CIM331 reference material (%) | Relative biological activity compared to main peak (%) | Features |
|---|---|---|---|---|
| Pre-Region 2 | b8 | 23±18 | 21±17 | Asu103 in HC |
| | b4+b5+b6+b7 | 89±5 | 80±4 | Asu103 in HC |
| Pre-Region 1 | b2+b3 | 73±12 | 66±11 | Asu103 in HC, LMWS (HH dimer, LL dimer (Hinge disulfide scrambling structure), etc.) |
| | b1 | 45±7 | 41±6 | IsoAsp in CDR (Asp99/102/103) in HC, LMWS |
| Post-Region | a1 | 110±6 | 100±6 | Deamidation at Asn384 in HC, LMWS (HHL-Cvs, L-Cys, etc.) |
| | a2+a3 | 110±7 | 100±6 | LMWS (HL), etc. |
| | a4 | 90±5 | 82±5 | Structural variant |
| | a5 | 88±6 | 79±6 | Deamidation at Asn55 in HC, HMWS |
| | a6 | 94±4 | 85±4 | Deamidation at Asn55 in HC, HMWS |

| | | | | |
|---|---|---|---|---|
| HC: heavy chain; LMWS: low molecular weight species; HMWS: high molecular weight species | | | | |

### [Example 2] Evaluation of aspartic acid isomerization variants (isoAsp forms and succinimide forms) in Pre-Region 2

The fractions containing each peak separated and obtained using the column and mobile phases described in Example 1 were diluted with a denaturant solution, pH 5.5, containing guanidium chloride, and then reduced with tris(2-carboxyethyl)phosphine. After buffer exchange with a digestion buffer, pH 5.5, containing 2-morpholinoethanesulfonic acid and urea, digestion reaction was performed using trypsin.

Formic acid was added to the resulting digestion reaction solution. This was injected into reverse-phase high-performance liquid chromatography for separation, and identification of peptides and calculation of modification ratios on peptide basis were carried out using a mass spectrometer. The ratio of a modification of interest on peptide basis was calculated from peak areas in the MS chromatogram, and this was determined to be the ratio of the variant of interest. Since it was found that the peak of the unmodified peptide overlapped with the peak of isoAsp residue-containing peptide on the MS chromatogram, modification ratios on peptide basis were calculated by the following formula: Modification ratio on peptide basis (%) = (peak area of modified peptide of interest*1)/{(peak area of modified peptide of interest) + (peak area of unmodified peptide corresponding to modified peptide of interest*2) - (peak area of isoAsp residue-containing peptide)} × 100
(* 1: referring to an isoAsp residue-containing peptide or a succinimide residue-containing peptide derived from an aspartic acid isomerization variant)
(*2: referring to an unmodified, native peptide which has the same amino acid sequence as the modified peptide except for the modified part in the modified peptide)

The result of analysis showed that a succinimide residue-containing peptide was detected at the highest level in the fraction containing peak b8, which is among the peaks of Pre-Region 2, of all AE-HPLC peaks. Specifically, in peak b8, alteration (modification) of Asp103 (Asp residue at Kabat numbering position 99) to a succinimide residue (Asu103) was detected at a ratio of 54.0%. On the other hand, the ratio of modification of Asp99 (Asp residue at Kabat numbering position 95) to a succinimide residue was low (2.7%). The modification of Asp102 (Asp residue at Kabat numbering position 98) to a succinimide residue and the modification of Asp99, Asp102, and Asp103 to an isoAsp residue were not detected. ···D⁹⁹GYDAsu¹⁰³G¹⁰⁴···

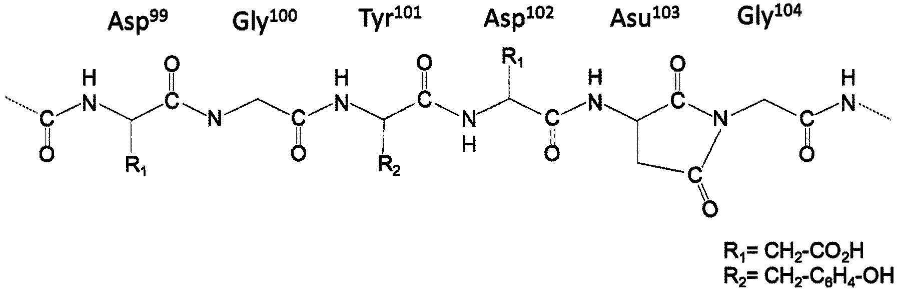

Thus, as compared to the main peak containing the main ingredient nemolizumab (Asu99 and Asu102: not detected, Asu103: 3.4%), which was separated and obtained with the column and mobile phases as described in Example 1, it was found that aspartic acid isomerization variants (Asu forms) were eluted predominantly in Pre-Region 2.

### [Example 3] Evaluation of aspartic acid isomerization variants (isoAsp forms and succinimide forms) in Pre-Region 1

Similarly to Example 2, the fractions containing each peak separated and obtained using the column and mobile phases described in Example 1 were diluted with a denaturant solution, pH 5.5, containing guanidium chloride, and then reduced with tris(2-carboxyethyl)phosphine. After buffer exchange with a digestion buffer, pH 5.5, containing 2-morpholinoethanesulfonic acid and urea, digestion reaction was performed using trypsin.

Formic acid was added to the resulting digestion reaction solution. This was injected into reverse-phase high-performance liquid chromatography for separation, and identification of peptides and calculation of modification ratios on peptide basis were carried out using a mass spectrometer. The ratio of a modification of interest on peptide basis was calculated from peak areas in the MS chromatogram, and this was determined to be the ratio of a variant of interest. Since it was found that the peak of the unmodified peptide overlapped with the peak of isoAsp residue-containing peptide on the MS chromatogram, modification ratios on peptide basis were calculated by the following formula: Modification ratio on peptide basis (%) = (peak area of modified peptide of interest*1)/{(peak area of modified peptide of interest) + (peak area of unmodified peptide corresponding to modified peptide of interest*2) - (peak area of isoAsp residue-containing peptide)} × 100
(*1: referring to an isoAsp residue-containing peptide or a succinimide residue-containing peptide derived from an aspartic acid isomerization variant)
(*2: referring to an unmodified, native peptide which has the same amino acid sequence as the modified peptide except for the modified part in the modified peptide)

The theoretically possible isoAsp forms are the below three structures (the top shows the modification of Asp99 to an isoAsp residue, the middle shows the modification of Asp 102 to an isoAsp residue, and the bottom shows the modification of Asp103 to an isoAsp residue). However, it has not been identified which of these Asp sites is/are actually isomerized. This is because, although a structurally altered amino acid site can be identified by peptide mapping tandem mass spectrometry (MSMS analysis) if the structural alteration leads to an altered molecular weight, isoAsp has the same molecular weight as Asp, making the above-mentioned identification with MSMS difficult.

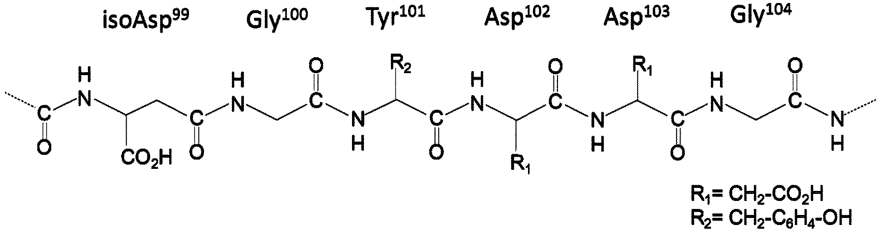

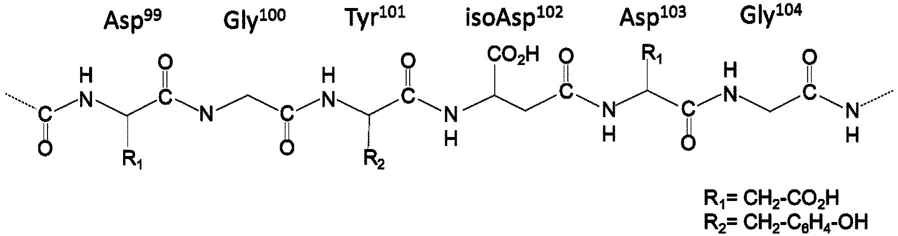

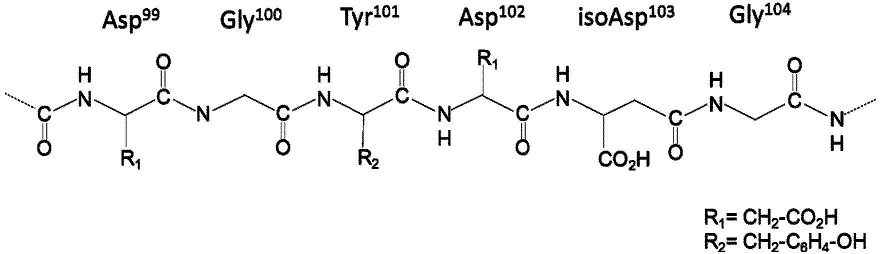

As a result of the analysis, isoAsp residue-containing peptides were detected at the highest level (two different isoAsp residue-containing peptides at 11.6% and 2.7%) in the fraction containing peak b1, which is among the peaks of Pre-Region 1, of all AE-HPLC peaks. Meanwhile, in peak b1, the ratio of alteration of Asp99 to a succinimide residue (Asu99) was 1.1%, that of alteration of Asp 102 to a succinimide residue (Asu102) was 0.2%, and that of alteration of Asp103 to a succinimide residue (Asu103) was 4.7%.

Thus, it was found that, as compared to the main peak containing the main ingredient nemolizumab (the ratio of modification to an isoAsp residue at any of Asp99, Asp102, and Asp103 is 0.5%) which was separated and obtained with the column and mobile phases as described in Example 1, aspartic acid isomerization variants (isoAsp forms) were eluted predominantly in Pre-Region 1.

### [Example 4] Evaluation of low molecular weight species (LMWS) in Pre-Region 1

### [4.1] Evaluation using non-reduced LC-ESI-MS

The fractions containing each peak separated and obtained with the column and mobile phases described in Example 1 were diluted with 0.1% formic acid, then injected into high performance liquid chromatography, desalted with a desalting column, and then subjected to identification of LMWS using a mass spectrometer.

As a result, it was identified that peaks b2 and b3, which are among the peaks of Pre-Region 1, contained the HH dimer and LL dimer.

### [4.2] Non-reduced tryptic peptide mapping evaluation

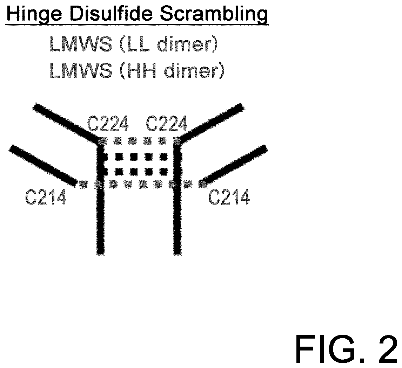

The fractions containing each peak separated and obtained using the column and mobile phases described in Example 1 were diluted with a denaturant solution, pH7.0, containing guanidium chloride, for denaturation treatment. This was followed by capping of cysteine side chains with iodoacetic acid, buffer exchange with a digestion buffer, pH7.0, containing Tris, urea, and EDTA, and digestion reaction with trypsin. Formic acid was added to the resulting digestion reaction solution. This was injected into reverse-phase high-performance liquid chromatography for separation, and identification of peptides was carried out using a mass spectrometer. As a result, it was identified that the HH dimer had a structure in which heavy chain C224, which normally forms a disulfide bond with light chain C214, linked the two heavy chains with each other. The LL dimer was presumed to have a structure in which light chain C214, which normally forms a disulfide bond with heavy chain C224, linked the two light chains with each other (Fig. 2).

### [4.3] Non-reduced CE-SDS evaluation

The fractions containing each peak separated and obtained using the column and mobile phases described in Example 1 were buffer-exchanged with 0.1 mol/L sodium phosphate buffer, then mixed with an SDS solution containing N-ethylmaleimide, and heated for denaturation and capping of cysteine side chains. Next, a dye reaction solution containing potassium cyanide and FQ-dye was added to the solution, and heated for labeling with the fluorescent dye. An SDS solution was added to the resulting solution, and analyzed by capillary electrophoresis-SDS.

As a result, in peaks b2 and b3, the content of the HH dimer and the LL dimer was 18.4 CPA (corrected peak area)% and 8.6 CPA%, respectively.

### [Example 5] Evaluation of biological activity of aspartic acid isomerization variants (isoAsp forms and succinimide forms)

The biological activity of each variant was evaluated by measuring the activity of inhibiting the proliferation of BaF/hIL-31R cells (BaF cells expressing human IL-31R), which proliferate in an IL-31-dependent manner.

Specifically, FBS/RPMI medium containing IL-31 was added to the fractions containing each peak separated and obtained using the column and mobile phases described in Example 1 to prepare dilutions at various concentrations. Each dilution was added to each well of a 96-well microplate. After adding a suspension of BaF/hIL-31R cells to each well, the cells were cultured in a CO₂ incubator at 37°C for 24 hours. Next, filtered alamarBlue was added, and the cells were further cultured in a CO₂ incubator at 37°C for 3 hours. Then, absorbance (OD₅₇₀-OD₆₀₀) was measured using a microplate reader.

As a result, when the main peak was used as a baseline (when normalized to the main peak as 100%), the biological activity of peak b8 was 21% relative to the main peak, indicating that the biological activity of the succinimide form predominantly contained in peak b8 (Asu103 variant which amounted to 54.0%) was significantly reduced.

It was also shown that the biological activity of peak b1 (isoAsp form content: 11.6%) was reduced to 41% relative to the main peak. It is strongly suggested that the Asp isomerization in this isoAsp form affects the biological activity because it is a chemical change in the CDRs. (Harris RJ. Heterogeneity of recombinant antibodies: linking structure to function. Dev Biol (Basel) 2005; 122:117-27; Dick, LWJ, et al, Identification and measurement of isoaspartic acid formation in the complementarity determining region of a fully human monoclonal antibody. J Chromatogr B Analyt Technol Biomed Life Sci, 2009;877:3841-9)

### [Example 6] Confirmation that AE-HPLC serves as a control/analysis method for aspartic acid isomerization variants (isoAsp forms and succinimide forms)

In the purification process of a nemolizumab-containing solution obtained by culturing of nemolizumab-expressing host cells, in order to examine the effect of the hold times of the antibody-containing solution after:
the harvesting step by centrifugation/filtration after production culture (HCCFs), and
the subsequent protein A affinity chromatography purification step (affinity pools) and before each next step (sample hold time after each purification step) on the content of aspartic acid isomerization variants (isoAsp forms and succinimide forms), the content of isoAsp forms and succinimide forms was examined in various samples (HCCFs (23°C, after 0 or 72 hours of holding) and affinity pools (23°C, after 0, 24, 48, 72, or 138 hours of holding)). The content of the variants relative to the unmodified form was determined on peptide basis by, as in Examples 2 and 3, digestion of each sample with trypsin and separation by HPLC, followed by MS analysis for quantification using MS intensities. The content of each variant on peptide basis was calculated using the following formula: Content of variant on peptide basis (intensity %) = (MS intensity*2 of modified peptide of interest*1)/{(MS intensity of modified peptide of interest) + (MS intensity of unmodified peptide corresponding to modified peptide of interest*3)} × 100

(*1: referring to an isoAsp residue-containing peptide or a succinimide residue-containing peptide derived from an aspartic acid isomerization variant)
(*2: MS intensity is the intensity of a MS spectrum derived from the peptide.)
(*3: referring to an unmodified, native peptide which has the same amino acid sequence as the modified peptide except for the modified part in the modified peptide.)

The result showed that the aspartic acid isomerization variants (isoAsp forms and succinimide forms) increased significantly in the affinity pools in a time-dependent manner (Table 4).

In addition, the above samples (HCCFs (23°C, after 0 or 72 hours of holding) and affinity pools (23°C, after 0, 24, 48, 72, or 138 hours of holding)) were separated by AE-HPLC as in Example 1, and the area ratio of each region in the HPLC chromatogram was determined. The correlations between the peptide-basis content of isoAsp forms and the area ratio of Pre-Region 1 in AE-HPLC and between the peptide-basis content of succinimide forms and the area ratio of Pre-Region 2 in AE-HPLC obtained by these analyses were examined. As a result, the isoAsp form content was found to correlate with the area ratio of Pre-Region 1 (Fig. 3). Also, the succinimide form content was found to correlate with the area ratio of Pre-Region 2 (Fig. 3), demonstrating that AE-HPLC serves as a control/analysis method for the content of these variants.

In addition, in the affinity pools, the area increase rate of Pre-Region 2 per hour was 0.0552 area%/h, and that of Pre-Region 1 was 0.0448 area%/h (Table 4). Given the high area increase rates per hour, appropriate setting of the sample hold time between the affinity chromatography purification step and the next step proved to be important for the quality assurance of nemolizumab.

**[Table 4]**

| DS lot | Process | Hold time (hour) | Total isoAsp (Intensity %) | Pre-Region 1 by AE-HPLC (area%) | Total Asu (Intensity %) | Pre-Region 2 by AE-HPLC (area%) |
|---|---|---|---|---|---|---|
| A | HCCF | 0 | 3.856 | 7.711 | 4.142 | 4.647 |
| | (23°C) | 72 | 4.150 | 7.849 | 4.755 | 4.412 |
| | Affinity pool (23°C) | 0 | 4.001 | 8.401 | 4.537 | 5.335 |
| | | 24 | 4.055 | 9.709 | 5.744 | 6.822 |
| | | 48 | 4.569 | 11.083 | 5.917 | 8.351 |
| | | 72 | 5.813 | 12.186 | 7.053 | 10.690 |
| | | 138 | 6.787 | 15.340 | 9.270 | 13.665 |
| B | HCCF (23°C) | 0 | 3.492 | 7.851 | 4.249 | 5.130 |
| | | 72 | 3.729 | 8..165 | 4.391 | 4.728 |
| | Affinity pool (23°C) | 0 | 4.390 | 8.954 | 5.018 | 6.210 |
| | | 24 | 4.643 | 10.092 | 5.189 | 7.564 |
| | | 48 | 5.022 | 11.509 | 5.604 | 8.827 |
| | | 72 | 5.695 | 12.189 | 7.625 | 9.844 |
| | | 138 | 8.237 | 15.529 | 10.245 | 13.234 |
| C | HCCF (23°C) | 0 | 3.839 | 7.746 | 4.454 | 5.231 |
| | | 72 | 3.729 | 7.919 | 2.930 | 5.134 |
| | Affinity pool (23°C) | 0 | 4.049 | 8.507 | 5.096 | 5.880 |
| | | 24 | 4.481 | 9.823 | 5.146 | 7.316 |
| | | 48 | 5.437 | 11.132 | 6.250 | 8.748 |
| | | 72 | 5.982 | 11.949 | 6.179 | 9.632 |
| | | 138 | 6.853 | 15.416 | 9.343 | 13.397 |

### [Example 7] Method for suppressing generation of aspartic acid isomerization variants (isoAsp forms and succinimide forms) in drug substance manufacture

### [7.1] Pre-Region 1

The shelf-life specification of nemolizumab drug products was set to 19.3% or lower for the AE-HPLC area ratio of Pre-Region 1, based on the predicted effects on clinical performance, biological activity, PK, safety, and immunogenicity. IsoAsp forms are common modified forms found in antibody pharmaceuticals, and considered to have low effect on safety and immunogenicity. For biological activity, peak b1 was found to have as low as 45% biological activity relative to the CIM331 reference material (nemolizumab) (Table 3). However, an increase in the AE-HPLC area ratio of Pre-Region 1 up to 19.3% was expected not to have significant impacts on the biological activity of the entire drug product.

Next, from the long-term stability test of the nemolizumab drug product, the amount of increase of Pre-Region 1 in 36 months of storage was estimated. As a result, the upper limit of the 95% confidence interval for the slope obtained with respect to the storage period (M) and the area ratio of Pre-Region 1 (area%) was 0.154 area%/M. Thus, the increase of the area ratio in 36M was estimated to be 5.5 area% (0.154 area%/M x 36 M = 5.544 area%). Accordingly, the release specification of the drug substance was set to 13.8 area% (19.3%-5.5%) or less for this area ratio. In addition, as described above, the area increase rate of Pre-Region 1 per hour in affinity pools was 0.0448 area%/h (Table 4), which is the highest area increase rate in the purification process; therefore, appropriate setting of the hold time of affinity pools proved to be important for quality assurance. From these stability results, it was found that in order to assure appropriate quality, it is preferred that the hold time of affinity pools (18°C-28°C) be within 24 hours (the increase range of 1.08 area%).

A regression line was obtained based on the data plotted with the peptide-basis content of isoAsp forms on the X-axis and the AE-HPLC area ratio of Pre-Region 1 on the Y-axis (Fig. 3) as shown above. The obtained regression line was used to calculate the total isoAsp form content on peptide basis corresponding to the area ratio of Pre-Region 1 of 19.3%. This calculation led to 9.4% as a ratio relative to the sum of the isoAsp forms and the corresponding unmodified peptide which has undergone no modification into isoAsp residues or Asu residues (the unmodified peptide which has the same amino acid sequence as the isoAsp forms except that the sites modified into isoAsp residues and Asu residues are aspartic acid). Based on this peptide-basis content, the theoretical maximum content of isoAsp forms on antibody basis^{*1} was calculated using the following binomial distribution-based formulas:
* 1: corresponding to when Asu forms are 0%.
Peptide-basis content: X (%) <9.4%>
isoAsp form with both heavy chains subjected to isomerization to isoAsp residues: X²
<(0.094)² × 100 = 0.00883 → 0.88%>
Native form with both heavy chains not modified: (1-X)² <(1-0.094) x (1-0.094) = 0.8208 → 82.1%>
isoAsp form with either heavy chain subjected to isomerization to isoAsp residues: X(1-X) + (1-X)X = 2X(1-X) <2 x 0.094 x (1-0.094) = 0.1703 → 17.0%>

Accordingly, the total isoAsp form content of 9.4% on peptide basis (the AE-HPLC area ratio of Pre-Region 1 of 19.3%) was found to be equivalent to the total isoAsp form content of 17.9% on antibody basis, which is the sum of the maximum content of isoAsp forms in which the two heavy chains contain isoAsp residues: 0.88%, and that of isoAsp forms in which one of the two heavy chains contains isoAsp residues: 17.0%.

### [7.2] Pre-Region 2

The shelf-life specification of nemolizumab drug products was set to 13.8% or lower for the AE-HPLC area ratio of Pre-Region 2, based on the predicted effects on clinical performance, biological activity, PK, safety, and immunogenicity. Asu forms are common modified forms found in antibody pharmaceuticals, and considered to have low effect on safety and immunogenicity. For biological activity, peak b8 was found to have as low as 23% biological activity relative to the CIM331 reference material (nemolizumab) (Table 3). However, an increase in the AE-HPLC area ratio of Pre-Region 2 up to 13.8% was expected not to have significant impacts on the biological activity of the entire drug product.

Next, from the long-term stability test of the nemolizumab drug product, the amount of increase of Pre-Region 2 in 36 months of storage was estimated. As a result, the upper limit of the 95% confidence interval for the slope obtained with respect to the storage period (M) and the area ratio of Pre-Region 2 (area%) was 0.123 area%/M. Thus, the increase of the area ratio in 36M was estimated to be 4.4 area% (0.123 area%/M x 36 M = 4.428 area%). Accordingly, the release specification of the drug substance was set to 9.4 area% (13.8%-4.4%) or less for this area ratio. In addition, as described above, the area increase rate of Pre-Region 2 per hour in affinity pools was 0.0552 area%/h (Table 4), which is the highest area increase rate in the purification process; therefore, appropriate setting of the hold time of affinity pools proved to be important for quality assurance. From these stability results, it was found that in order to assure appropriate quality, it is preferred that the hold time of affinity pools (18°C-28°C) be within 24 hours (the increase range of 1.32 area%).

A regression line was obtained based on the data plotted with the peptide-basis content of Asu forms on the X-axis and the AE-HPLC area ratio of Pre-Region 2 on the Y-axis (Fig. 3) as shown above. The obtained regression line was used to calculate the total Asu form content on peptide basis corresponding to the area ratio of Pre-Region 2 of 13.8%. This led to 9.8% as a ratio relative to the sum of the Asu forms and the corresponding unmodified peptide which has undergone no modification into isoAsp residues or Asu residues (the unmodified peptide which has the same amino acid sequence as the Asu forms except that the sites modified into isoAsp residues and Asu residues are aspartic acid). Based on this peptide-basis content, the theoretical maximum content of Asu forms on antibody basis^{*1} was calculated using the following binomial distribution-based formulas:
*1: corresponding to when isoAsp forms are 0%.
Peptide-basis content: X (%) <9.8%>
Asu form with both heavy chains subjected to isomerization to Asu residues: X² <(0.098)² × 100 = 0.00960 → 0.96%>
Native form with both heavy chains not modified: (1-X)² <(1-0.098) x (1-0.098) = 0.8136 → 81.4%>
Asu form with either heavy chain subjected to isomerization to Asu residues: 2X(1-X) <2 × 0.098 × (1-0.098) = 0.1767 → 17.7%>

Accordingly, the total Asu form content of 9.8% on peptide basis (the AE-HPLC area ratio of Pre-Region 2 of 13.8%) was found to be equivalent to the total Asu form content of 18.6% on antibody basis, which is the sum of the maximum content of Asu forms in which the two heavy chains contain Asu residues: 0.96%, and that of Asu forms in which one of the two heavy chains contains Asu residues: 17.7%.

### [Example 8] Evaluation of CDR deamidation forms in Post-Region

The fractions containing each peak separated and obtained using the column and mobile phases described in Example 1 were diluted with a denaturant solution containing guanidium chloride, pH7.0, and reduced with dithiothreitol. This was followed by capping of cysteine side chains with iodoacetic acid, buffer exchange with a digestion buffer, pH7.0, containing Tris, urea, and EDTA, and digestion reaction with trypsin. Formic acid was added to the resulting digestion reaction solution. This was injected into reverse-phase high-performance liquid chromatography for separation, and identification of peptides and quantification of variants were carried out using a mass spectrometer.

As a result, of all AE-HPLC peaks, the fraction containing peak a6, which is among the peaks of Post-Region, showed the highest deamidation level of 14.9% at Asn55 in the CDRs of the nemolizumab heavy chain. Since the deamidation level in this peak was low, the effect of deamidation at Asn55 on the biological activity could not be directly evaluated by biological activity measurement. However, as CDRs have an crucial role in antigen binding, it is strongly suggested that the deamiation at Asn55 in the CDRs affects the biological activity.
Harris, R.J., et al., Identification of multiple sources of charge heterogeneity in a recombinant antibody. J Chromatogr B Biomed Sci Appl, 2001. 752(2): p. 233-45
Yan B, Steen S, Hambly D, et al. Succinimide formation at Asn 55 in the complementarity determining region of a recombinant monoclonal antibody IgG1 heavy chain. J Pharm Sci 2009; 98:3509-21.
Qi P, Volkin DB, Zhao H, et al. Characterization of the photodegradation of a human IgG1 monoclonal antibody formulated as a high-concentration liquid dosage form. J Pharm Sci 2009; 98:3117-30

Furthermore, analysis of the drug substance stored at 40°C for 12 weeks by the same method showed that the above deamidation content was 1.6% on peptide basis even for the drug substance stored at 40°C for 12 weeks, and it was also presumed that the content would not significantly increase during the storage of drug products at 30°C (for example, 1.6% on peptide basis).

### [Example 9] Evaluation of LMWS in Post-Region

### [9.1] Evaluation using non-reduced LC-ESI-MS

The fractions containing each peak separated and obtained with the column and mobile phases described in Example 1 were diluted with 0.1% formic acid, then injected into high performance liquid chromatography, desalted with a desalting column, and then subjected to identification of LMWS using a mass spectrometer.

As a result, it was identified that the fraction containing peaks a2 and a3, which are among the peaks of Post-Region, contained the HL form, and the fraction containing peak a1 contained the cysteinylated light chain and the cysteinylated HHL form.

### [9.2] Evaluation using non-reduced tryptic peptide mapping

The fractions containing each peak separated and obtained using the column and mobile phases described in Example 1 were diluted with a denaturant solution containing guanidium chloride, pH7.0, for denaturation treatment. This was followed by capping of cysteine side chains with iodoacetic acid, buffer exchange with a digestion buffer, pH7.0, containing Tris, urea, and EDTA, and digestion reaction with trypsin. Formic acid was added to the resulting digestion reaction solution. This was injected into reverse-phase high-performance liquid chromatography for separation, and identification of peptides was carried out using a mass spectrometer.

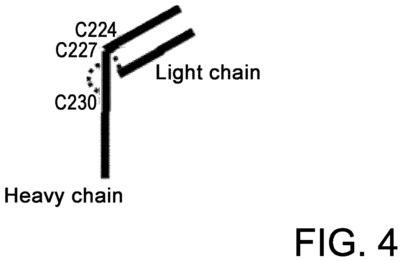

The results led to a conclusion that the HL form contained in peaks a2 and a3 had a structure in which Cys227 and Cys230 in one heavy chain form a disulfide bond (Fig. 4).

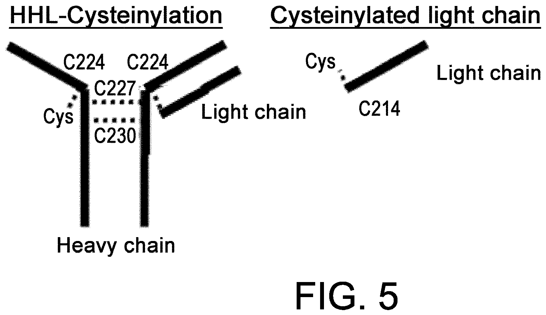

Meanwhile, the results did not lead to identification of the site of cysteinylation in the cysteinylated light chain and the cysteinylated HHL form contained in peak a1, but these were presumed to have the structures shown in Fig. 5.

### [9.3] Evaluation using non-reduced CE-SDS

The fractions containing each peak separated and obtained using the column and mobile phases described in Example 1 were buffer-exchanged with 0.1 mol/L sodium phosphate buffer, and then mixed with an SDS solution containing N-ethylmaleimide for capping of cysteine side chains. Next, a dye reaction solution containing potassium cyanide and FQ-dye was added to the solution, and labeling with the fluorescent dye was carried out. An SDS solution was added to the resulting solution, and analyzed by capillary electrophoresis-SDS.

As a result, it was determined that the content of the HL form in the fraction containing peaks a2 and a3 was 16.7 CPA%, and the content of the cysteinylated light chain in the fraction containing peak a1 was 14.5 CPA%.

### [Example 10] Control of LMWS

### [10.1] Method of evaluating low molecular weight species (CE-SDS)

A drug substance and drug product of nemolizumab were each buffer-exchanged with 0.1 mol/L sodium phosphate buffer, and then mixed with an SDS solution containing N-ethylmaleimide for capping of cysteine side chains. Next, a dye reaction solution containing potassium cyanide and FQ-dye was added to the solution, and labeling with the fluorescent dye was carried out. An SDS solution was added to the resulting solution, and subjected to separation using capillary electrophoresis-SDS (CE-SDS). A group of peaks with shorter migration times than the main component of the nemolizumab drug substance and drug product (nemolizumab) was designated as a group of peaks with smaller molecular weights than nemolizumab. This peak group was determined to be the subject of evaluation by the LMWS control/analysis method.

### [10.2] Control of low molecular weight species content (CE-SDS)

The shelf-life specification of nemolizumab drug products was set to 11.0 CPA% or lower for the content of LMWS as analyzed by CE-SDS (including LMWS identified in the fraction containing peaks b2 and b3 and the fraction containing peaks a1, a2, and a3 according to AE-HPLC), based on the predicted effects on clinical performance, biological activity, PK, safety, and immunogenicity. As the LMWS content was not found to increase in the stability test of the drug product, the release specification of the drug substance and the drug product was also set to 11.0 CPA% or lower.

### [Industrial Applicability]

The present inventors discovered antibody variants with lower biological activity than nemolizumab, and methods of analysis thereof. The antibody variants and analysis methods of the present invention are useful for quality assessment of drug substances and drug products of nemolizumab, and also useful in developing nemolizumab drug products with reduced contents of the antibody variants of the present invention, and in developing methods for suppressing generation of the antibody variants of the present invention. In addition, the pharmaceutical compositions of the present invention containing nemolizumab with reduced contents of the antibody variants of the present invention are useful as means to treat and/or prevent atopic dermatitis, dialysis pruritus, and other pruritus.

## Claims

1. A variant of an antibody having a variable region comprising the amino acid sequence DGYDDGPYTLET (SEQ ID NO: 3), wherein
(a) the amino acid residue D at position 1 from the N-terminus of the above sequence,
(b) the amino acid residue D at position 4 from the N-terminus of the above sequence, or
(c) the amino acid residue D at position 5 from the N-terminus of the above sequence
is a succinimide residue or an isoaspartic acid residue.

2. The antibody variant of claim 1, wherein the sequence is a CDR sequence.

3. The antibody variant of claim 1, wherein the sequence is a sequence contained in a heavy chain.

4. The antibody variant of claim 1, wherein the antibody having a variable region comprising the amino acid sequence DGYDDGPYTLET (SEQ ID NO: 3) is:
(1) an antibody comprising a heavy chain variable region comprising CDR1 set forth in SEQ ID NO: 1, CDR2 set forth in SEQ ID NO: 2, and CDR3 set forth in SEQ ID NO: 3, and a light chain variable region comprising CDR1 set forth in SEQ ID NO: 4, CDR2 set forth in SEQ ID NO: 5, and CDR3 set forth in SEQ ID NO: 6;
(2) an antibody comprising a heavy chain variable region set forth in SEQ ID NO: 7 and a light chain variable region set forth in SEQ ID NO: 8; or
(3) an antibody comprising a heavy chain set forth in SEQ ID NO: 9 and a light chain set forth in SEQ ID NO: 10.

5. The antibody variant of any one of claims 1-4, wherein the antibody having a variable region comprising the amino acid sequence DGYDDGPYTLET (SEQ ID NO: 3) is nemolizumab.

6. The antibody variant of any one of claims 1-5, which has a shorter retention time than the antibody having a variable region comprising the amino acid sequence DGYDDGPYTLET (SEQ ID NO: 3) when separated using anion exchange chromatography.

7. The antibody variant of any one of claims 1-6, which has lower biological activity than the antibody having a variable region comprising the amino acid sequence DGYDDGPYTLET (SEQ ID NO: 3).

8. A pharmaceutical composition comprising the antibody variant of any one of claims 1-7.

9. A pharmaceutical composition comprising the antibody variant of any one of claims 1-7, wherein the ratio of an isoaspartic acid residue-containing antibody variant in the pharmaceutical composition to the sum of the antibody variant and the unmodified antibody is 36% or less, and/or, after enzymatic digestion, the ratio of an isoaspartic acid residue-containing peptide derived from the antibody variant to the sum of the isoaspartic acid residue-containing peptide and the corresponding unmodified peptide is 20% or less.

10. A pharmaceutical composition comprising the antibody variant of any one of claims 1-7, wherein when the pharmaceutical composition is separated using anion exchange chromatography, the ratio of the peak area containing an isoaspartic acid residue-containing antibody variant to the total peak area is 40.3 area% or less.

11. A pharmaceutical composition comprising the antibody variant of any one of claims 1-7, wherein the ratio of a succinimide residue-containing antibody variant in the pharmaceutical composition to the unmodified antibody is 36% or less, and/or, after enzymatic digestion, the ratio of a succinimide residue-containing peptide derived from the antibody variant to the sum of the succinimide residue-containing peptide and the corresponding unmodified peptide is 20% or less.

12. A pharmaceutical composition comprising the antibody variant of any one of claims 1-7, wherein when the pharmaceutical composition is separated using anion exchange chromatography, the ratio of the peak area containing an isoaspartic acid residue-containing antibody variant to the total peak area is 29.1 area% or less.

13. The pharmaceutical composition of any one of claims 8-12, which comprises nemolizumab.

14. A method for detecting the antibody variant of any one of claims 1-7, comprising the step of separating a sample comprising an antibody having a variable region comprising the amino acid sequence DGYDDGPYTLET (SEQ ID NO: 3) or the antibody that has been enzymatically treated, by affinity chromatography, ion exchange chromatography, normal-phase chromatography, reverse-phase chromatography, hydrophilic interaction chromatography (HILIC), hydrophobic interaction chromatography (HIC), charge-based separation, size exclusion chromatography (SEC), gel permeation chromatography (GPC), or a combination thereof.

15. The method of claim 14, wherein the ion exchange chromatography is anion exchange chromatography.

16. The method of claim 15, wherein the anion exchange chromatography uses a column with an inner diameter of 4.6-7.5 mm, a length of 35-150 mm, and a particle diameter of 2.5-10 µm at a column temperature of 25-50°C and performs elution with a mobile phase of pH6.0-10.0.

17. The method of any one of claims 14-16, which, following the step of separating a sample comprising the antibody that has been enzymatically treated, comprises the step of quantifying a peptide by mass spectrometry and/or UV absorption measurement.

18. A method for producing a composition comprising nemolizumab, comprising the step of performing the method of any one of claims 14-17.

19. A method for producing a composition comprising nemolizumab, comprising the step of purifying a solution containing nemolizumab by affinity chromatography, ion exchange chromatography, hydrophilic interaction chromatography (HILIC), hydrophobic interaction chromatography (HIC), charge-based separation, size exclusion chromatography (SEC), gel permeation chromatography (GPC), or a combination thereof.

20. The method of claim 19, wherein the purifying step comprises purifying a composition comprising nemolizumab obtained from a nemolizumab-producing cell by affinity chromatography.

21. The method of claim 20, wherein the hold time of the nemolizumab-containing solution after the purification by affinity chromatography is 72 hours or less, and the content of the antibody variant of any one of claims 1-7 in the composition is lower than when the hold time is longer.

22. A method for reducing the content of the antibody variant of any one of claims 1-7, comprising the step of purifying an antibody-containing composition obtained from a cell producing an antibody having a variable region comprising the amino acid sequence DGYDDGPYTLET (SEQ ID NO: 3) by affinity chromatography.

23. The method of claim 22, wherein the hold time of the nemolizumab-containing solution after the purification by affinity chromatography is 72 hours or less, and the content of the antibody variant of any one of claims 1-7 in the composition is lower than when the hold time is longer.

24. An antibody variant of nemolizumab, which has a structure formed by two heavy chains of nemolizumab which are linked with each other via cysteines at EU numbering position 224 in the heavy chains.

25. An antibody variant of nemolizumab, which has a structure formed by two light chains of nemolizumab which are linked with each other via cysteines at EU numbering position 214 in the light chains.

26. An antibody variant of nemolizumab, which has a structure formed by one light chain and one heavy chain of nemolizumab, wherein a cysteine at EU numbering position 214 in the light chain is linked with a cysteine at EU numbering position 224 in the heavy chain, and cysteines at EU numbering positions 227 and 230 in the heavy chain are linked within the single heavy chain.

27. An antibody variant of nemolizumab, which has a structure formed by one light chain, wherein a cysteine at EU numbering position 214 in the light chain of nemolizumab is linked with a free cysteine.

28. An antibody variant of nemolizumab, which has a structure formed by two heavy chains and one light chain of nemolizumab, wherein the two heavy chains are linked with each other via cysteines at two positions: EU numbering positions 227 and 230; a cysteine at EU numbering position 224 in the first heavy chain is linked with a cysteine at EU numbering position 214 in the light chain, and a cysteine at EU numbering position 224 in the second heavy chain is linked with a free cysteine.

29. A pharmaceutical composition comprising the antibody variant of any one of claims 24-28, wherein the ratio of the antibody variant in the total antibody molecules in the pharmaceutical composition is 11.0 CPA% or less.

30. An antibody variant of nemolizumab, wherein asparagine at Kabat numbering position 55 in a heavy chain of nemolizumab is deamidated.

31. A pharmaceutical composition comprising the antibody variant of claim 30, wherein the ratio of a deamidation-containing peptide derived from the antibody variant after enzymatic digestion of the pharmaceutical composition to the sum of the deamidation-containing peptide and the corresponding unmodified peptide is 2.0% or less.
